# EUROPEAN PATENT APPLICATION

(11) **EP 4 268 823 A1**
(43) Date of publication of application: **01.11.2023**
(21) Application number: 22169820.2
(22) Date of filing: 25.04.2022
(51) Int. Cl.: A61K 31/517, A61K 31/551, A61P 25/00, A61P 25/28

(54) **COMPOUND FOR TREATING NEUROINFLAMMATION**

(71) Applicant: Universitätsklinikum Hamburg-Eppendorf, 20246 Hamburg (DE)
(72) Inventor: FRIESE, Manuel, 20251 Hamburg (DE); ENGLER, Jan Broder, 20251 Hamburg (DE); ROTHAMMER, Nicola, 20149 Hamburg (DE)
(74) Representative: Uexküll & Stolberg

(57) **Abstract**

The invention relates to a compound which is effective in inhibiting the function of the histone methyltransferase G9a and the use of such compound in treating or preventing the progression of Multiple Sclerosis in a subject. The invention further relates to a compound which is effective in inhibiting the function of the histone methyltransferase G9a and the use of such compound in a method of reducing or ameliorating ferroptosis in a MS patient. The invention also provides a pharmaceutical composition comprising a compound which is effective in inhibiting the function of the histone methyltransferase G9a. The invention further relates to *in vitro* methods for identifying pharmaceutically active compounds that are useful for treating or preventing the progression of Multiple Sclerosis in a subject.

## Description

The invention relates to a compound which is effective in inhibiting the function of the histone methyltransferase G9a and the use of such compound in treating or preventing the progression of Multiple Sclerosis in a subject. The invention further relates to a compound which is effective in inhibiting the function of the histone methyltransferase G9a and the use of such compound in a method of reducing or ameliorating ferroptosis in a MS patient. The invention also provides a pharmaceutical composition comprising a compound which is effective in inhibiting the function of the histone methyltransferase G9a. The invention further relates to *in vitro* methods for identifying pharmaceutically active compounds that are useful for treating or preventing the progression of Multiple Sclerosis in a subject.

### BACKGROUND OF THE INVENTION

Neuroinflammation is involved in several neurological and psychiatric diseases, in which activation of brain-resident microglia (1) and infiltration of peripheral immune cells cause neuronal stress and injury (2). As a prototypic neuroinflammatory disease, multiple sclerosis (MS) is characterized by inflammatory lesions that cause damage to the central nervous system (CNS) and lead to neuronal dysfunction and clinical impairment (3). Although inflammatory activity declines over the course of MS, persistent low-grade inflammation (4) and inflammation-independent progression of neurodegeneration lead to continuous worsening of disability (5). In this regard, chronic progressive MS is reminiscent of primary neurodegenerative diseases that are accompanied by low grade inflammation (6). However, at the stage of progressive neurodegeneration, immunosuppressive treatments that are effective in the early inflammatory phase of MS mostly lose their efficacy. Therefore, to deliver long-term benefits to patients suffering from neuroinflammation, novel neuroprotective therapeutics with direct action on neurons are urgently needed (7).

Neurodegeneration in MS has been attributed to the generation of reactive oxygen and nitrogen species (8), mitochondrial dysfunction with impaired energy production (9), and glutamate excitotoxicity that causes influx of calcium into neurons (10) resulting in ionic dyshomeostasis and cell death (2, 5). Similar pathways have been picked up in recent single cell transcriptomics analysis of MS postmortem brain samples. Affected neurons showed a strong upregulation of signatures associated with oxidative stress, mitochondrial dysfunction, and cell death pathways (11). However, the extraction of master regulators of neuronal maladaptation remains challenging (12). As postmortem studies are inherently complicated by the fact that available samples mostly represent late chronic stages of the disease, the neuronal stress response to inflammation was investigated under tightly controlled conditions in the MS animal model experimental autoimmune encephalomyelitis (EAE). To generate a comprehensive inventory of neuronal adaptation during inflammatory stress, translating ribosome affinity purification of spinal cord motor neurons that are directly affected in EAE (13) was exploited. As part of this study, inflammation-induced dysregulation of neuronal proteostasis with toxic aggregation of the presynaptic protein bassoon was discovered, and a therapeutic approach to clear aggregates by proteasome activation was devised (13). This complements other strategies to ameliorate inflammatory neurodegeneration by modulation of mitochondrial function (14), neuronal ion channels (15-18) or glutamate receptors (19-21). However, besides the pharmacological appeal of targeting specific molecular pathways, neurodegeneration emerges as a multimodal process with many detrimental cascades running in parallel (22). Therefore, shutting down the underlying maladaptive neuronal stress response as a whole may provide greater and more consistent benefit, as it depends less on specific pathways and their pathological contribution in a given situation.

Despite the progress that has been made in recent years, uncovering the driving forces behind progressive neurodegeneration in MS remains a major scientific challenge. It is an object of the present invention to identify compounds which can be used to effectively treat MS or prevent the progression of MS in a subject, preferably a human subject. It is also an object of the present invention to identify compounds that reinstall neuronal homeostasis and rebalance stress responses.

### DESCRIPTION OF THE INVENTION

The present invention is based on the insight that the histone methyltransferase G9a is strongly induced in neurons during neuroinflammation in EAE mice and MS patients. It was furthermore found that the histone methyltransferase G9a strongly influences neuronal gene expression in a way that promotes ferroptosis, neuronal loss and clinical disability. Specifically, G9a promotes ferroptosis via the epigenetic repression of anti-ferroptotic genes resulting in neuronal loss during inflammatory neurodegeneration. More importantly, it was found that a robust neuroprotective effect can be achieved in neuronal cultures and during CNS inflammation *in vivo* by inhibition of the histone methyltransferase G9a. These findings reveal G9a as a stress-induced epigenetic amplifier of neuronal ferroptosis and highlight G9a inhibition as a neuroprotective therapy.

In light of the above findings, the present invention specifically contemplates to use antagonists and/or inhibitors of the histone methyltransferase G9a for treating MS or preventing the progression of MS in a subject. Compounds that inhibit the function of G9a have been described in the art. However, the use of such compounds for treating MS has not been proposed.

Thus, in a first aspect the invention relates to a compound which is effective in inhibiting the function of the methyltransferase G9a for use in a method of treating or preventing the progression of Multiple Sclerosis in a subject. In a second aspect, the invention relates to a compound which is effective in inhibiting the function of the methyltransferase G9a for use in a method of reducing or ameliorating ferroptosis in a MS patient. In a third aspect, the invention relates to a compound which is effective in inhibiting the function of the methyltransferase G9a for use in a method of ferroptosis-mediated neuronal loss in a MS patient.

The MS to be treated can be any form of MS, including relapsing-remitting MS (RRMS), primary progressive MS (PPMS), and secondary progressive MS (SPMS). In a preferred embodiment, the MS to be treated is relapsing-remitting MS.

The subject to be treated with the G9a-inhibitory compound will normally be a mammal, and preferably is a human. Generally, the subject can be of any age. The subject preferably suffers from MS, more preferably from relapsing-remitting MS. The subject may be in any stage of the disease, i.e. the disease may be in an early stage wherein the subject shows only the first pathological signs that are normally associated with MS, or the subject may be in a late stage of the disease. The subject to be treated may have a genetic or other predisposition for developing MS.

The inhibitory compounds of the invention effectively prevent damage and/or loss of neurons in the nervous system of a subject, preferably in the central nervous system (CNS). As used herein, the CNS is to be understood to comprise the brain and the spinal cord. In a preferred embodiment, the compounds of the invention are administered for preventing damage and/or loss of neurons in the brain. In a further embodiment, the compounds of the invention are administered for preventing damage and/or loss of neurons in the spinal cord. According to the invention, progressive damage and/or loss of neurons may be halted by administration of the compounds of the invention. Halting the damage and/or the loss of neurons means that the pathological processes of the neurodegenerative disease which finally result in damage and/or loss of neurons are stopped or at least reduced.

### Inhibitory compounds

The compounds of the present invention are pharmaceutically active compounds which are effective in decreasing the expression and/or the activity of the methyltransferase G9a. The compounds can include all different types of organic or inorganic molecules, including peptides, polypeptides, oligo- or polysaccharides, fatty acids, steroids, and the like. Typically, the compounds will be small molecules with a molecular weight of less than about 2,500 daltons, less than 2000 daltons, less than 1500 daltons, less than 1000 daltons, or less than 550 daltons. In a particularly preferred embodiment of the invention the compounds which inhibit the function of methyltransferase G9a have a molecular weight of less than about 550 daltons.

Preferably, the compounds of the present invention are able to cross the blood-brain barrier, i.e. they are blood-brain barrier-permeable. This means that the compounds are able to reach a site in the brain which is affected by neuronal injury and/or loss. Inhibitory compounds with a molecular weight of below 550 Daltons are particularly suitable, since they are often able to cross the blood-brain barrier, or their transport across the blood-brain barrier is achieved by other means, such as by transporters or by hydrophobic stretches in the compound which allow diffusion across the blood-brain barrier.

The compounds contemplated by the invention affect the function of a methyltransferase G9a protein. In a preferred embodiment, the inhibitory compounds interfere with the human histone methyltransferase G9a protein. In the context of the present invention, the terms "histone methyltransferase G9a", "methyltransferase G9a" or "G9a" refer to the epigenetic regulator euchromatic histone lysine *N*-methyltransferase-2, an enzyme which is encoded by the gene *Ehmt2* gene. G9a catalyzes the dimethylation of lysine 9 on core histone 3 (H3K9me2), wherein said dimethylation results in local transcriptional repression. The human methyltransferase G9a protein occurs in four different isoforms. The nucleotide sequences of the isoforms are depicted in SEQ ID NOs:1-4. The corresponding protein sequences of the isoforms are depicted in SEQ ID NOs:5-8.

Preferably, the compounds contemplated by the present invention inhibit the function of a methyltransferase G9a comprising or consisting of the amino acid sequence depicted in SEQ ID NOs:5-8 or an amino acid sequence having at least 90% sequence identity to the sequence of SEQ ID NOs:5-8. In another preferred embodiment, the compounds contemplated by the invention inhibit the function of a methyltransferase G9a comprising or consisting of the amino acid sequence depicted in SEQ ID NOs:5-8 or an amino acid sequence having at least 90% sequence identity to the sequence of SEQ ID NOs:5-8.

The compounds which are contemplated herein for use in the treatment of MS are effective in inhibiting the function of the methyltransferase G9a. This means that the compounds effectively reduce the extent of dimethylation of lysine 9 on core histone 3 (H3K9me2). In a preferred embodiment, the compound decreases the G9a activity by at least 10%, 20%, 30%, 40%, 50%, 60%, 70%, 80%, 90%, or 95% compared to the activity in the absence of the compound. As used herein, the reduction of the G9a activity is understood as the prevention of a full dimethylation of lysine 9 on core histone 3. In a particularly preferred embodiment, the inhibitory compound completely blocks the G9a activity such that there is no detectable methylation or dimethylation activity of core histone 3.

A decrease in the activity of the methyltransferase G9a, i.e. its methylation activity, may be determined by any suitable assay. For example, G9a activity can be measured in a cell culture system by determining the H3K9me2 level after application of an inhibitor via immunocytochemistry staining. Fluorescence of the H3K9me2 signal can be analyzed by confocal microscopy or by measuring the fluorescence in a microplate reader. In addition, suitable assays are sold by several manufacturers, for example the Histone H3 Panel (mono methyl K9, dimethyl K9, tri methyl K9) (ab113754) of abcam (Cambridge, UK) and the G9a Chemiluminescent Assay Kit of BPSBioscience (San Diego, USA).

Preferably, the inhibitory compound is specific for the methyltransferase G9a, i.e. the compound inhibits the expression and/or activity of G9a, while it does essentially not inhibit the expression and/or activity of other proteins or enzymes, e.g. other methyltransferase. As a result of the G9a specificity, the compounds according to the invention elicit no or only tolerable side effects when administered to a subject.

The G9a-inhibitory compound of the invention can be derived from different groups of molecules. For example, inhibitory compounds can include antisense polynucleotides which bind to the gene encoding G9a and block transcription, RNA processing and/or translation of said gene. The antisense molecules can be RNA or DNA molecules. Also, the G9a-inhibitory compound of the invention can be a RNA molecule which exerts its effect by RNA interference. Examples for such compounds are RNAi molecules and siRNA molecules that are capable of blocking translation of the G9a-encoding mRNA. Alternatively, the inhibitory compound of the invention can be a ribozyme that cleaves the G9a-encoding mRNA. Other classes of molecules which may give rise to suitable G9a-inhibitory compounds include peptides, antibodies and antibody fragments. Peptides that bind and interfere with the G9a protein may be conveniently screened in random peptide libraries.

A number of inhibitors of G9a have been described in the art. These inhibitors include UNC0642 (45), BIX01294 (45), UNC0224 (45), UNC0321 (45), E72 (45), UNC0638 (45), BRD9539 (45), UNC1479 (45), CSV0C018875 (89), A-366 (90), UNC0646 (87), UNC0631 (87), HKMTI-1-247 (87), HKMTI-1-248 (87), CM-272 (87), BRD4770 (87), CBC-12 (87), DCG066 (87), CPUY074001 (87), CPUY074020 (87), TM2-115 (87), 867750 (87), 867751 (87), DS79932728 (88), and EZM-8266 (88).

In a preferred embodiment of the invention, the compound which is effective in inhibiting the function of the histone methyltransferase G9a and used for treating or preventing the progression of Multiple Sclerosis is UNC0642 (2-(4,4-difluoropiperidin-1-yl)-6-methoxy-N-[1-(propan-2-yl)piperidin-4-yl]-7-[3-(pyrrolidin-1-yl)propoxy]quinazolin-4-amine; CAS Number 1481677-78-4) or a pharmaceutically acceptable salt, solvate, tautomer or ester thereof. Examples of pharmaceutically acceptable salts of UNC0642 include hydrochlorides and sulphates. The structure of UNC0642 is as follows:

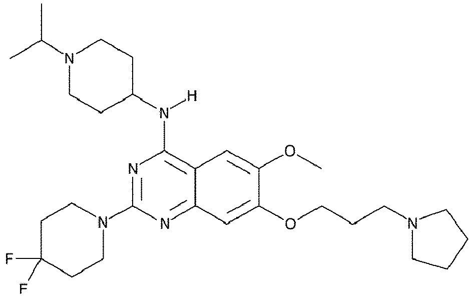

The above structure can be substituted or otherwise modified at one or more sites, as long as these modifications do not substantially impair the inhibitory effect of UNC0642 and do not result in any undesired toxic side effects. For example, one or more hydrogen atoms of the C-H bonds of the heterocyclic ring system can be substituted with halogen atoms, such as chlorine, bromine or iodine atoms. Further, the hydrogen of the C-H bonds can also be replaced by an alkyl group, such as methyl, ethyl or propyl.

The pharmaceutical compositions of the present invention will comprise an amount of the G9a-inhibitory compound that is effective to inhibit G9a activity, thereby reducing the extent of neurodegeneration by protecting the CNS from neuronal loss. The therapeutically effective amount of the G9a-inhibitory compound to be administered will depend on several parameters, such as the mode of administration, the particular type of MS disease, the severity of the disease, and the age, height, weight, health, and physical condition of the individual to be treated. A therapeutically effective amount of the G9a-inhibitory compound can be determined by one of ordinary skill in the art without undue experimentation given the disclosure set forth herein.

The G9a-inhibitory compound will preferably be administered to the subject to be treated, e.g. the subject suffering from MS, in an amount that ranges from 0.1 µg/kg body weight to 10,000 µg/kg body weight, e.g. from 0.5 µg/kg to 7,500 µg/kg body weight, from 1.0 µg/kg to 5,000 µg/kg body weight, from 5.0 µg/kg to 3,000 µg/kg body weight, from 7.5 µg/kg to 2,500 µg/kg body weight, from 10 µg/kg to 2,000 µg/kg body weight, from 25 µg/kg to 1,500 µg/kg body weight, from 50 µg/kg to 1,000 µg/kg body weight, from 100 µg/kg to 800 µg/kg body weight, from 300 µg/kg to 600 µg/kg body weight, and more preferably from 400 µg/kg to 500 µg/kg body weight.

### Pharmaceutical compositions

The G9a-inhibitory compound of the present invention will normally be provided in the form of a pharmaceutical composition. Therefore, in a fourth aspect, the invention relates to a pharmaceutical composition comprising a compound which is effective in inhibiting the function of the euchromatic histone lysine N-methyltransferase-2 (G9a) for use in a method of treating or preventing the progression of Multiple Sclerosis in a subject. In a fifth aspect, the invention relates to a pharmaceutical composition comprising a compound which is effective in inhibiting the function of the euchromatic histone lysine N-methyltransferase-2 (G9a) for use in a method of reducing or ameliorating ferroptosis in a Multiple Sclerosis patient.

The pharmaceutical compositions of the invention normally comprise one or more excipients, carriers and/or diluents suitable for the intended way of administration. Generally, the G9a-inhibitory compound may be administered in any suitable form that does not interfere with its G9a-inhibitory activity. For example, the compound may be administered orally in the form of tablets, capsules, granule, powder, liquids, and the like. Alternatively, the G9a-inhibitory compound may be formulated for being administered parenterally, e.g. by intravenous injection or intravenous infusion. In a preferred aspect, the G9a-inhibitory compound is administered to the subject by intravenous infusion, more preferably by short-term infusion within less than 60 min, e.g. within 30 min, 20 min or 15 min.

Compositions suitable for injection and/or infusion include solutions or dispersions and powders for the extemporaneous preparation of such injectable solutions or dispersions. The composition for injection must be sterile and should be stable under the conditions of manufacturing and storage. Preferably, the compositions for injection and/or infusion also include a preservative, such as a chlorobutanol, phenol, ascorbic acid, thimerosal, and the like. For intravenous administration, suitable carriers may comprise physiological saline, bacteriostatic water, Cremophor EL^{™} (BASF) or phosphate-buffered saline (PBS). Sterile solutions for injection and/or infusion can be prepared by incorporating the G9a-inhibitory compound in the required amount in an appropriate solvent followed by filter sterilization.

Apart from the G9a-inhibitory compound, the pharmaceutical composition provided by the present invention may further comprise other anti-neurodegenerative or anti-inflammatory compounds which are commonly used in the treatment of MS, such as interferon beta, glatiramer acetate, teriflunomide, cladribine, fampridine, fingolimod, ozanimod, ponesimod, siponimod, alemtuzumab, natalizumab, ocrelizumab, ofatumumab or rituximab. Where the G9a-inhibitory compound is used in combination with another anti-neurodegenerative agent, the two active ingredients can be administered to the subject in the form of a single pharmaceutical composition comprising both agents and pharmaceutically acceptable excipients and carriers. Administration of such a pharmaceutical composition will automatically result in a simultaneous administration of both agents. Alternatively, the two therapeutic agents may also be administered separately from each other, i.e. in the form of two separate pharmaceutical compositions, one containing the G9a-inhibitory compound, and the other containing the additional anti-neurodegenerative or anti-inflammatory agent. The two separate compositions can be administered simultaneously, i.e. at the same time at two distinct sites of administration, or they may be administered sequentially (in either order) to the same site or to different sites of administration.

Preferably, both the composition comprising the G9a-inhibitory compound and the composition comprising the additional anti-neurodegenerative or anti-inflammatory agent are administered according to a weekly dosing regimen, more preferably a regimen in which a single dose of the G9a-inhibitory compound and a single dose of the anti-neurodegenerative or anti-inflammatory agent is administered every week for a treatment period of 2 or more weeks, for example, 3, 4, 5, 6, 7, 8, 9, 10, 11, 12, 13, 14, 15, 16, 17, 18, 19, 20, or more weeks. It will also be possible to administer the overall weekly dose of the G9a-inhibitory compound and/or the anti-neurodegenerative or anti-inflammatory agent in more than one administration per week, e.g. in 2 or 3 administrations per week. In a preferred embodiment, the amount of the G9a-inhibitory compound to be administered weekly is delivered as a single intravenous infusion per week, and the amount of the anti-neurodegenerative or anti-inflammatory agent to be administered weekly is also given as a single intravenous infusion per week, either at the same day of administration of the G9a-inhibitory compound, e.g. within 6-12 hours after administration of the G9a-inhibitory compound has been completed.

### In vitro screening methods

The insight that a decreased expression of the gene encoding G9a and/or a decreased activity of the methyltransferase G9a prevents the progression of MS in a subject allows the design of screening assays which identify pharmaceutically active compounds which are effective in treating and/or preventing of MS. Thus, the present invention provides methods for identifying a pharmaceutically active compound that could be used for treating or preventing MS. The methods of the invention can be used for drug screening approaches that aim to identify new pharmaceutically active for treating MS.

Accordingly, in a sixth aspect, the invention relates to an *in vitro* method for identifying a pharmaceutically active compound for treating Multiple Sclerosis or preventing the progression of Multiple Sclerosis in a subject, comprising
(a) contacting a candidate compound with a functional methyltransferase G9a;
(b) detecting whether said candidate compound interferes with the function of the methyltransferase G9a;
wherein a compound which inhibits the function of the methyltransferase G9a is considered suitable for treating Multiple Sclerosis or preventing the progression of Multiple Sclerosis in a subject.

A decrease in the activity of the methyltransferase G9a, i.e. its methylation activity, may be determined by any suitable assay. For example, G9a activity can be measured in a cell culture system by determining the H3K9me2 level after application of an inhibitor via immunocytochemistry staining. Fluorescence of the H3K9me2 signal can be analyzed by confocal microscopy or by measuring the fluorescence in a microplate reader. In addition, suitable assays are sold by several manufacturers, for example the Histone H3 Panel (mono methyl K9, dimethyl K9, tri methyl K9) (ab113754) of abcam (Cambridge, UK) and the G9a Chemiluminescent Assay Kit of BPSBioscience (San Diego, USA).

In an alternative approach, the screening can target compounds which interfere with G9a transcription and/or translation. The invention therefore relates, in a seventh aspect, to an *in vitro* method for identifying a pharmaceutically active compound for treating Multiple Sclerosis or preventing the progression of Multiple Sclerosis in a subject, comprising
(a) contacting a candidate compound with a cell that expresses a functional methyltransferase G9a;
(b) detecting whether said candidate compound decreases the transcription of the methyltransferase G9a gene and/or the translation of the methyltransferase G9a mRNA;
wherein a compound which decreases the transcription of the methyltransferase G9a gene and/or the translation of the methyltransferase G9a mRNA is suitable for treating or preventing the progression of Multiple Sclerosis.

Screening methods which monitor the transcription of the gene encoding G9a and/or the translation of the G9a mRNA are particularly useful, e.g. for identifying compounds which inhibit the G9a by decreasing G9a expression. By use of these methods, it will for example be possible to identify compounds that target transcription factors which are relevant for G9a expression. Also, suitable ribozymes or antisense DNA molecules can be identified in this way.

A number of different screening methods to evaluate the effects of candidate compounds on gene expression or enzyme activity may be used. The methods of the invention make use of any suitable method to detect a decrease in the expression level of the gene encoding G9a and/or in the activity of G9a. Suitable methods include e.g. biochemical or cellular methods. The skilled person would be able to identify such methods with his or her common knowledge.

For example, a decrease in the expression level of the gene encoding G9a, i.e. the *Ehmt2* gene, may be detected by methods which allow the quantification of the transcription or the translation product of the gene encoding G9a, e.g. the quantification of G9a mRNA or protein. Such methods are well known in the art. For example, polymerase chain reaction (PCR)-based methods, such as standard PCR, real-time PCR and quantitative real-time PCR, or Northern Blots may be used to determine the concentration of the G9a mRNA. The concentration will be determined relative to a sample which is not contacted with the respective candidate compound, i.e. relative to a negative control, or to the same sample before contacting it with the respective compound. The concentration of G9a protein may analogously be determined with standard methods such as SDS-PAGE, enzyme linked immunosorbent assays (ELISA), Western Blots, mass spectrometry or any other suitable means. The G9a protein concentration will then be compared to a sample which has not been contacted with the respective compound or to the same sample before contacting it with the respective compound.

The cell that expresses the functional G9a enzyme is preferably a mammalian cell, more preferably a human cell. The cell can be derived from a cell line, preferably a neuron- or brain cell-derived cell line, or from a primary cell culture, more preferably from a primary neuronal cell culture.

The *in vitro* methods of the present invention may preferably be carried out as high throughput screening techniques which allow for the examination of thousands of different compounds in a short period of time. Candidate compounds for use in screening methods can be provided in the form of libraries which comprise a high number of synthetic or natural compounds. High throughput screening techniques are described in detail in the prior art. The compounds identified by the screening methods may be validated in animal models, such as mouse models to confirm their activity *in vivo.*

The candidate compounds used in the screening methods of the invention can include all different types of molecules as described for the compounds of the present invention above.

If the assay uses a G9a protein, the protein can be one that has been recombinantly expressed. Preferably, the G9a protein used in the assay comprises the amino acid sequences set out in SEQ ID NO:5, 6, 7, or 8. However, it will be understood by the skilled person that the above assays can be performed not only with the specific sequences depicted in SEQ ID NO:5, 6, 7, or 8, but also with variants, derivatives and enzymatically active fragments of these G9a protein sequences. As used herein, variants of G9a are polypeptides that differ by one or more amino acid exchanges from the sequence shown in SEQ ID NOs:5-8. Generally, any amino acid residue of the amino acid sequence shown in SEQ ID NOs:5-8 can be exchanged for a different amino acid, provided the resultant sequence of the variant is still capable of catalyzing dimethylation of lysine 9 on core histone 3 (H3K9me2). In particular, variants may differ from their reference sequence shown in SEQ ID NOs:5-8 in up to 5%, 10%, 15%, or 20% of the amino acids. Polypeptides in which one or more amino acids were inserted in the amino acid sequence of SEQ ID NOs:5-8 are also included as variants. Such insertions can be made at any position of the polypeptide shown in SEQ ID NOs:5-8 as long as the variant is still able to catalyze dimethylation of H3K9me2. Moreover, polypeptides in which one or more amino acids are missing in comparison with SEQ ID NOs:5-8 are also considered to be variants of the polypeptides of SEQ ID NOs:5-8. Such deletions can apply to any amino acid position of the sequence of SEQ ID NOs:5-8.

Variants of the G9a protein will preferably have at least 80% sequence identity, more preferably at least 85% sequence identity, and even more preferably at least 90% sequence identity, e.g. 91%, 92%, 93%, 94%, 95%, 96%, 97%, 98%, 99% sequence identity, with the human G9a protein shown in SEQ ID NOs:5-8 when these sequences are optimally arranged, e.g. with the computer program GAP or BESTFIT using the default gap method. Computer programs for determination of the amino acid identity are known to the skilled person.

Enzymatically active fragments of a G9a methyltransferase may also be used in the above assay. In particular, the assay may be conducted with enzymatically active fragments of the sequence shown in SEQ ID NOs:5-8 or variants thereof. As used herein, enzymatically active fragments of the sequence shown in SEQ ID NOs:5-8 refer to peptides or polypeptides that differ from the amino acid sequence shown in SEQ ID NOs:5-8 or from the above-defined variants thereof by the absence of one or more amino acids at the N-terminus and/or at the C-terminus of the peptide or polypeptide.

Derivatives of the polypeptides shown in SEQ ID NOs:5-8 or of the variants thereof refer to polypeptides that possess amino acids with structural modifications, for example, modified amino acids, relative to a polypeptide shown in SEQ ID NOs:5-8 or variants thereof. These modified amino acids can be, e.g. amino acids that have been altered by phosphorylation, glycosylation, acetylation, thiolation, branching and/or cyclization. It is preferred that the variants or derivatives of the G9a polypeptide shown in SEQ ID NOs:5-8 or the active fragments of this polypeptide or its variants retain at least 75%, and preferably up to 80%, 85%, 90% or even up to 99% of the dimethylation activity of the G9a polypeptide shown in SEQ ID NOs:5-8.

In another aspect, the invention relates to the use of: (a) a polypeptide comprising the amino acid sequence shown in SEQ ID NOs:5-8 or a variant thereof having at least 80%, more preferably at least 90% sequence identity to the amino acid sequence shown in SEQ ID NOs:5-8; or (b) a polynucleotide encoding a polypeptide of (a) or the complement thereof for identifying pharmaceutically active compounds for treating MS or preventing the progression of MS.

### DESCRIPTION OF THE FIGURES

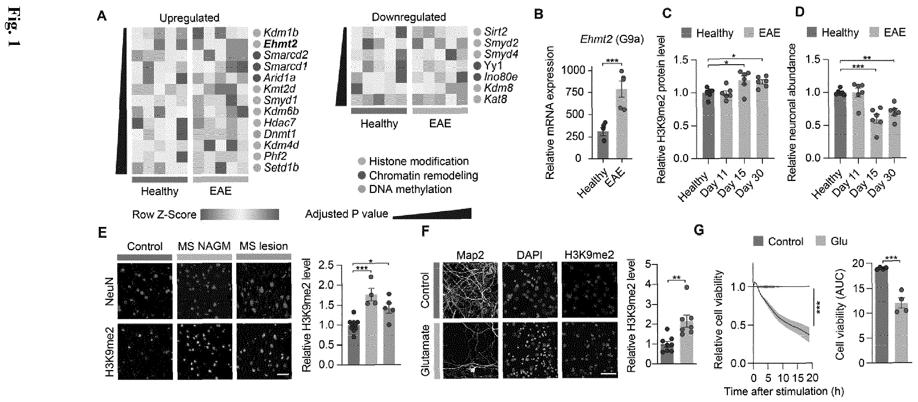

**Figure 1** shows that G9a-depedent H3K9me2 is induced by inflammation and excitotoxicity. (A) Gene expression heat map of top up- and downregulated epigenetic modifiers in inflamed motor neurons during EAE. Candidates were identified by DESeq2 and adjusted for multiple comparison by FDR (n = 5 biologically independent samples per group each pooled from 3 mice, log2(fold change) > 1; FDR-adjusted P < 0.05). (B) Normalized RNA-Seq expression of Ehmt2 in healthy and inflamed motor neurons. Statistical testing by DESeq2, using negative binomial generalized linear models adjusted for multiple comparisons by FDR; n = 5 biologically independent samples per group, each pooled from 3 mice, motor neurons versus inflamed motor neurons. (C) Flow cytometry of H3K9me2 immunofluorescence in spinal cord neuronal nuclei from healthy and EAE mice 11, 15, or 30 days after immunization (one-way ANOVA: n = 6 per group, F(3,20) = 5.802, P = 0.0051; Dunnett's post hoc test: Healthy vs. day 11 P = 0.9961; healthy vs. day 15 P = 0.0164; healthy vs. day 30 P = 0.0421). (D) Percentage of neuronal loss in spinal cord nuclei from healthy and EAE mice 11, 15, or 30 days after immunization (one-way ANOVA: n = 6 per group, F(3,20) = 14.90, P < 0.0001; Dunnett's post hoc test: Healthy vs. day 11 P > 0.9999; healthy vs. day 15 P < 0.0001; healthy vs. day 30 P = 0.0019). (E) Representative images and quantification of mean fluorescence of immunohistochemistry stainings from H3K9me2 in NeuN+ cells of brain sections from multiple sclerosis (MS) normal appearing grey matter (NAGM) or cortical MS lesions in comparison to control individuals without neurological diseases (one-way ANOVA: Controls, n = 10; MS NAGM, n = 4; MS lesion, n = 5, F(2,16) = 17.23, P = 0.0001; Tukey's post hoc test: Control vs. NAGM P = 0.0001; control vs. MS P = 0.0105; NAGM vs. MS P = 0.0997). Scale bar, 80 µm. (F) Representative image and quantification of H3K9me2 staining 8 hours after 20 µM glutamate stimulation (two-tailed Student's t-test, n = 8 control vs. n = 7 Glu, P = 0.0033). Scale bar, 50 µm. (G) Cell viability assay of cortical neurons stimulated with 20 µM glutamate, time course (left; two-way ANOVA; n = 4 independent experiments; F(1,6) = 45.30; P = 0.0005) and area under the curve (right; two-tailed Student's t-test n = 4 independent experiments; P = 0.0031) 19 hours after glutamate application. Data are shown as mean values ± s.e.m. ^{∗}P < 0.05, ^{∗∗}P < 0.01, ^{∗∗∗}P < 0.001.

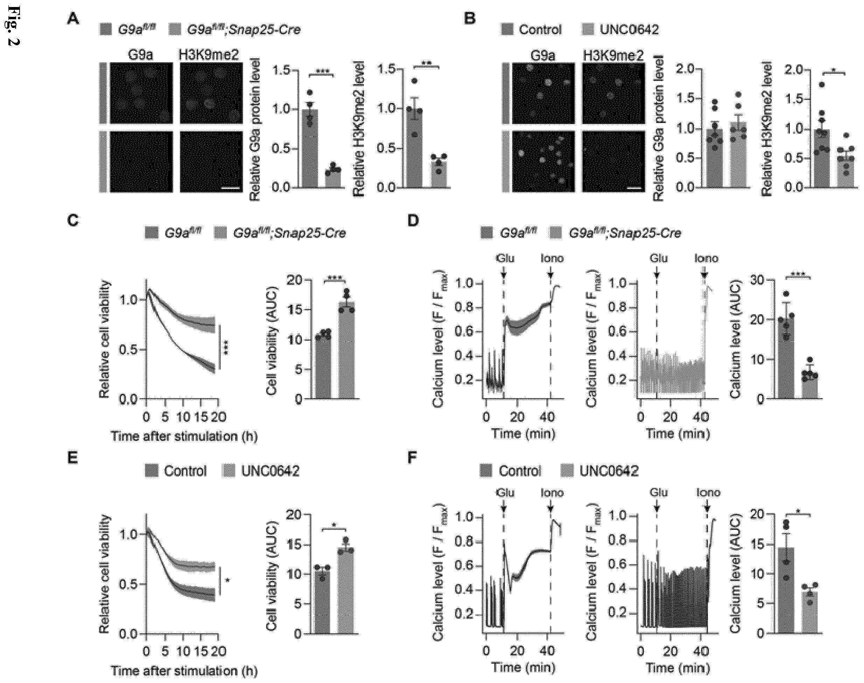

**Figure 2** shows that G9a promotes neuronal damage during glutamate-induced excitotoxicity. (A and B) Representative images (left) and quantification (right) of immunofluorescence stainings of G9a and H3K9me2 from G9afl/fl;Snap25-Cre (A; two-tailed Student's t-test, n = 4 independent experiments, G9a: P = 0.0002; H3K9me2: P = 0.0037) or UNC0642 pre-treated primary neuronal cultures compared to respective controls (B; two-tailed Student's t-test, G9a: DMSO n = 7, UNC0642 n = 6, P = 0.5479; H3K9me2: DMSO n = 8, UNC0642 n = 7, P = 0.0164). Scale bars, 20 µm (C) Time course (left; two-way ANOVA; n = 4 independent experiments; F(1,6) = 38.19; P < 0.0001) and quantification of the area under the curve (right; two-tailed Student's t-test, n = 4 independent experiments, P = 0.0008) of cell viability from G9afl/fl vs. G9afl/fl;Snap25-Cre primary cortical cultures. (D) Cytosolic calcium recordings of glutamate-exposed G9afl/fl and G9afl/fl;Snap25-Cre neuronal cultures (two-tailed Student's t-test, n = 5 independent experiments, P = 0.0001). (E) Time course (left; two-way ANOVA; n = 3 independent experiments; F(1,4) = 19.37; P = 0.0117) and quantification of the area under the curve (right; two-tailed Student's t-test, n = 3 independent experiments, P = 0.0115) of cell viability from DMSO- vs. UNC0642-treated primary cortical cultures. (F) Cytosolic calcium recordings of glutamate-exposed neuronal cultures treated with DMSO or UNC0642 (two-tailed Student's t-test, n = 4 independent experiments, P = 0.0193). Data are shown as mean values ± s.e.m. ^{∗}P < 0.05, ^{∗∗}P < 0.01, ^{∗∗∗}P < 0.001.

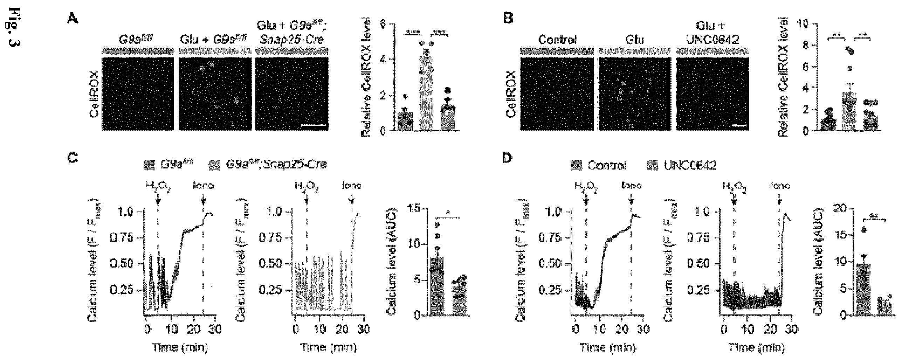

**Figure 3** shows that G9a potentiates oxidative stress in neurons. (A) Representative images (left) and quantification (right) of immunostaining of oxidative stress with CellROX green reagent in G9afl/fl vs. G9afl/fl;Snap25-Cre cultures (one-way ANOVA: n = 5 independent experiments, F(2,12) = 36.59, P < 0.0001, Tukey's post hoc test: Control vs. G9afl/fl + Glu, P < 0.0001; control vs. G9afl/fl;Snap25-Cre + Glu, P = 0.3929; G9afl/fl + Glu vs. G9afl/fl;Snap25-Cre + Glu, P < 0.0001); Scale bar, 40 µm. (B) Representative images (left) and quantification (right) of CellROX green reagent in DMSO- vs. UNC0642-treated cultures (one-way ANOVA: n = 10 independent experiments, F(2,27) = 8.614, P = 0.0013. Tukey's post hoc test: Control vs. Glu, P = 0.0017; control vs. UNC0642 + Glu, P = 0.7783; Glu vs. UNC0642 + Glu, P = 0.0093). Scale bar, 40 µm. (C) Cytosolic calcium recordings of H2O2-exposed G9afl/fl and G9afl/fl;Snap25-Cre neuronal cultures (two-tailed Student's t-test, n = 6 independent experiments, P = 0.0315). (D) Cytosolic calcium recordings of H2O2-exposed neuronal cultures treated with DMSO or UNC0642 (two-tailed Student's t-test, n = 5 independent experiments, P = 0.0062). Data are shown as mean values ± s.e.m. ^{∗}P < 0.05, ^{∗∗}P < 0.01, ^{∗∗∗}P < 0.001.

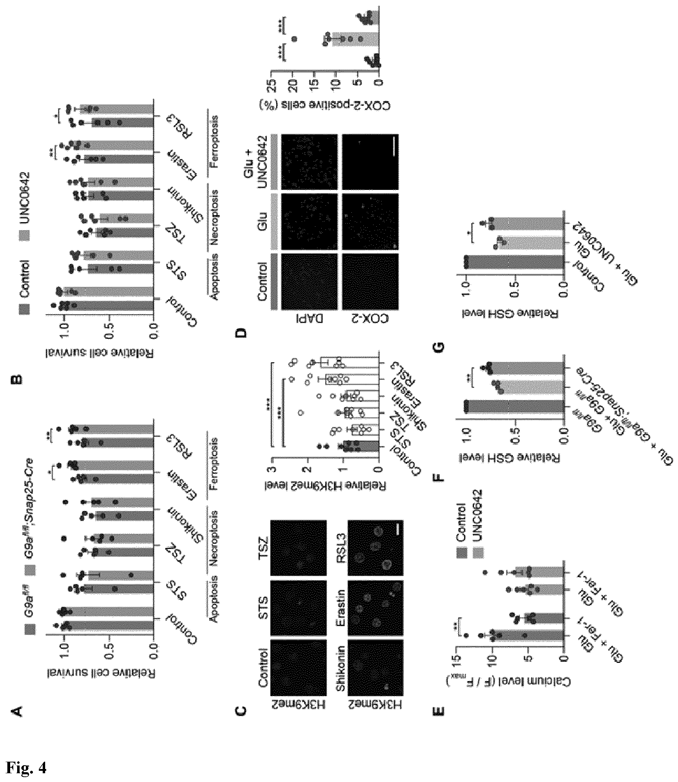

**Figure 4** shows that G9a induces neuronal cell death via ferroptosis. (A) Quantification of dead neurons with CellTox green reagent in response to induction of different cell death pathways in G9afl/fl vs. G9afl/fl;Snap25-Cre cultures (two-tailed paired Student's t-test, n = 5 independent experiments; Control: P > 0.999; STS: P = 0.4885; TSZ: P = 0.8128; Shikonin: P = 0.2156; Erastin: P = 0.0140; RSL3: P = 0.0043). (B) Quantification of dead neurons with CellTox green reagent in response to induction of different cell death pathways in cultures ± UNC0642 (two-tailed paired Student's t-test, n = 6 independent experiments; Control: P > 0.999; STS: P = 0.3786; TSZ: P = 0.4790; Shikonin: P = 0.8992; Erastin: P = 0.0039; RSL3: P = 0.0498). (C) Immunofluorescence of H3K9me2 in primary cortical neurons after treatment with indicated compounds (repeated measures one-way ANOVA: n = 8 independent experiments, F(5,35) = 26.91, P < 0.0001. Dunnett's post hoc test: Control. vs. STS, P = 0.0752; control. Vs. TSZ, P = 0.9952, control vs. Shikonin, P = 0.9327, control. vs. erastin, P < 0.0001, control. vs. RSL3, P < 0.0001). (D) Representative images (left) and quantification (right) of COX-2 immunostaining after glutamate stimulation in neuronal cultures ± UNC0642 (one-way ANOVA: n = 7 independent experiments, F(2,18) = 20.31, P = 0.0001; Tukey's post hoc test: Control vs. Glu, P < 0.0001; control vs. UNC0642 + Glu, P = 0.4834; Glu vs. UNC0642 + Glu, P = 0.0004). Scale bar, 40 µm (E) Quantification of Fmax-normalized cytosolic calcium responses after glutamate treatment ± UNC0642. Cultures were stimulated 10 min prior to glutamate treatment with ferrostatin-1 (two-way ANOVA with Bonferroni's post hoc test, n = 6 per group; F(1,20) = 10.90, P = 0.0036; Bonferroni's post hoc test: DMSO + Glu vs. DMSO + Glu + Fer-1, P = 0.0043, UNC0642 + Glu vs. UNC0642 + Glu + Fer-1, P = 0.5290). (F,G) Glutathione measurement in G9afl/fl vs. G9afl/fl;Snap25-Cre (G; two-tailed Student's t-test, n = 3 independent experiments, P = 0.0042) or UNC0642-treated cortical neurons (H; two-tailed Student's t-test, n = 4 independent experiments, P = 0.0469) in response to glutamate. Data are normalized to untreated controls. Data are shown as mean values ± s.e.m. ^{∗}P < 0.05, ^{∗∗}P < 0.01, ^{∗∗∗}P < 0.001.

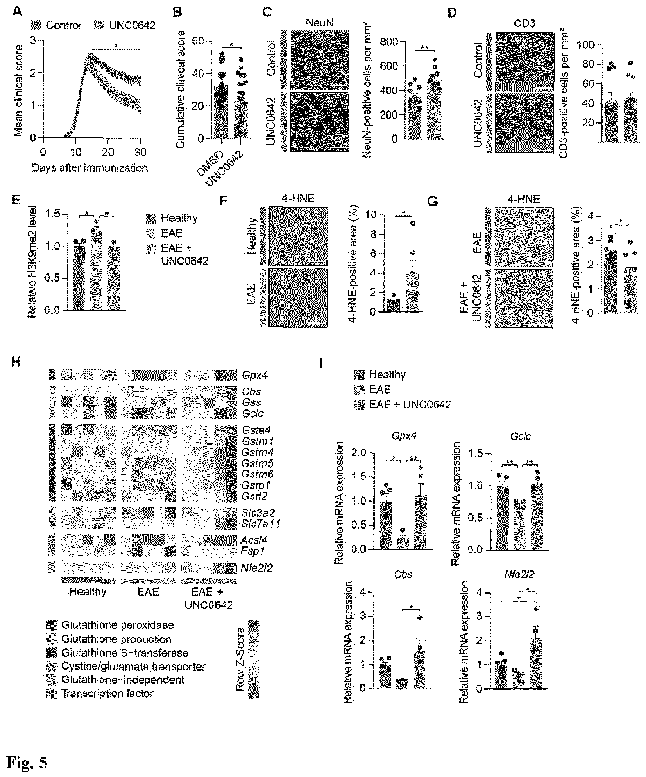

**Figure 5** shows that G9a amplifies neurodegeneration and represses anti-ferroptotic genes in CNS inflammation. (A) Disease course of mice subjected to EAE, which were treated with either DMSO or 5 mg kg-1 body weight UNC0642 beginning at disease onset. Data are pooled from two independent experiments. DMSO, n = 24; UNC0642, n = 23. (B) Quantification of cumulative EAE score from day 15 to day 30 (two-tailed Mann-Whitney U-test; DMSO, n = 24; UNC0642, n = 23; P = 0.0294). (C) Histopathological staining and quantification of neuronal loss in the spinal cord of EAE mice ± UNC0642 at day 30 (two-tailed Mann-Whitney U-test; n = 10 per group; P = 0.0068). Scale bars, 100 µm. (D) Histopathological staining and quantification of T cells (CD3) in the spinal cord of EAE mice ± UNC0642 at day 30 (two-tailed Mann-Whitney U-test; n = 10 per group; P = 0.7959). Scale bars, 100 µm. (E) Flow cytometry of H3K9me2 immunofluorescence of spinal cord neuronal nuclei from healthy, EAE, and UNC0642-treated EAE mice (one-way ANOVA: n = 4 mice each group, F(2,9) = 6.836, P = 0.0156. Tukey's post hoc test: Healthy vs. EAE, P = 0.0433; healthy vs. EAE + UNC0642, P = 0.8436; EAE vs. EAE + UNC0642, P = 0.0181). (F) Representative images and quantification of anti-4-HNE staining of cervical spinal cord sections from healthy vs. EAE mice during the acute phase (two-tailed Mann-Whitney U-test; n = 6 animals per group, P = 0.0043). Scale bars, 100 µm. (G) Representative images and quantification of cervical spinal cord section from chronic EAE treated with vehicle or 5 mg kg-1 body weight UNC0642 (two-tailed Mann-Whitney U-test; n = 12 animals per group, P = 0.0279). Scale bars, 100 µm. (H) Gene expression heatmap of qPCR data from sorted spinal cord neuronal nuclei of healthy, EAE, and UNC0642-treated EAE mice. n = 5 mice per group. (I) mRNA expression data from selected genes of healthy, EAE, and UNC0642-treated EAE mice (one-way ANOVA: Gpx4: healthy n = 5, EAE n = 4, EAE + UNC0642 n = 5, F(2,11) = 7.635, P = 0.0083. Tukey's post hoc test: Healthy vs. EAE, P = 0.0249; healthy vs. EAE + UNC0642, P = 0.8107; EAE vs. EAE + UNC0642, P = 0.0091; Cbs: Healthy n = 5, EAE n = 5, EAE + UNC0642 n = 4 F(2,11) = 5.838, P = 0.0187. Tukey's post hoc test: Healthy vs. EAE, P = 0.1490; healthy vs. EAE + UNC0642, P = 0.3473; EAE. vs. EAE + UNC0642, P = 0.0156; Gclc: Healthy n = 5, EAE n = 5, EAE + UNC0642 n = 5, F(2,12) = 11.92, P = 0.0014. Tukey's post hoc test: Healthy vs. EAE, P = 0.0047; healthy vs. EAE + UNC0642, P = 0.9021; EAE vs. EAE + UNC0642, P = 0.0022; Nfe212: Healthy n = 5, EAE n = 4, EAE + UNC0642 n = 4, F(2,10) = 7.420, P = 0.0106. Tukey's post hoc test: Healthy vs. EAE, P = 0.6056; healthy vs. EAE + UNC0642, P = 0.0384; EAE vs. EAE + UNC0642, P = 0.0107). Data are shown as mean values ± s.e.m. ^{∗}P < 0.05, ^{∗∗}P < 0.01, ^{∗∗∗}P < 0.001.

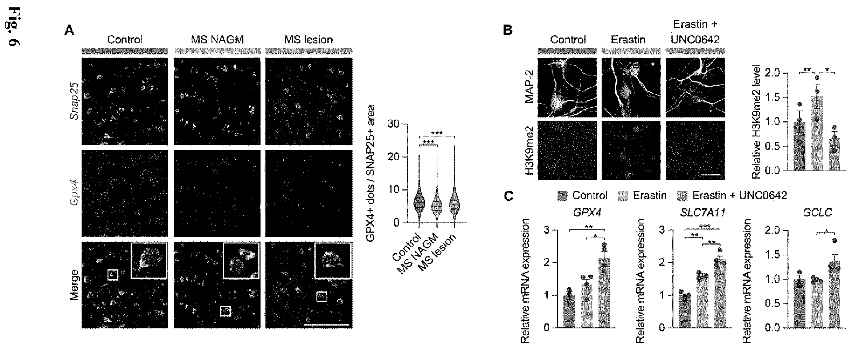

**Figure 6** shows that anti-ferroptotic GPX4 is suppressed in MS and induced by G9a inhibition (A) RNAScope fluorescence in situ hybridization of GPX4 transcripts in brain sections of control individuals and multiple sclerosis NAGM and cortical lesions (one-way ANOVA: Control n = 99,202 neurons; MS NAGM n = 229,718 neurons; MS lesion n = 131,294 neurons; F(2,460212) = 8445, P < 0.0001, Dunnett's post hoc test: Control vs. MS NAGM P < 0.0001, Control vs. MS lesion P < 0.0001). Scale bar, 100 µm. (B) H3K9me2 immunostaining and quantification in response to ferroptosis induction with erastin in hiPSC neurons (repeated measures one-way ANOVA: n = 3 independent experiments, F(1.060,2.12) = 49.21, P = 0.0169. Tukey's post hoc test: Control vs. + Erastin, P = 0.0082; control. vs. Erastin + UNC0642, P = 0.1136, Erastin vs. Erastin + UNC0642, P = 0.0327). Scale bar, 40 µm. (C) mRNA expression levels of indicated genes from hiPSC-neurons treated with erastin ± UNC0642 vs. Control (one-way ANOVA: GPX4: Control n = 3, Erastin n = 4, Erastin + UNC0642 n = 4, F(2,8) = 11.43, P = 0.0045. Tukey's post hoc test: Control vs. Erastin, P = 0.4265; Control vs. Erastin + UNC0642, P = 0.0048; Erastin vs. Erastin + UNC0642, P = 0.0211; SLC7A11: Control n = 4, Erastin n = 3, Erastin + UNC0642 n = 4, F(2,8) = 56.75, P < 0.0001. Tukey's post hoc test: Control vs. Erastin, P = 0.0015; Control vs. Erastin + UNC0642, P < 0.0001; Erastin vs. Erastin + UNC0642, P = 0.0059; GCLC: Control n = 3, Erastin n = 4, Erastin + UNC0642 n = 4, F(2,8) = 5.039, P = 0.0383. Tukey's post hoc test: Control vs. Erastin, P = 0.9897; Control vs. Erastin + UNC0642, P = 0.0816; Erastin vs. Erastin + UNC0642, P = 0.0487). Data are shown as mean values ± s.e.m. ^{∗}P < 0.05, ^{∗∗}P < 0.01, ^{∗∗∗}P < 0.001.

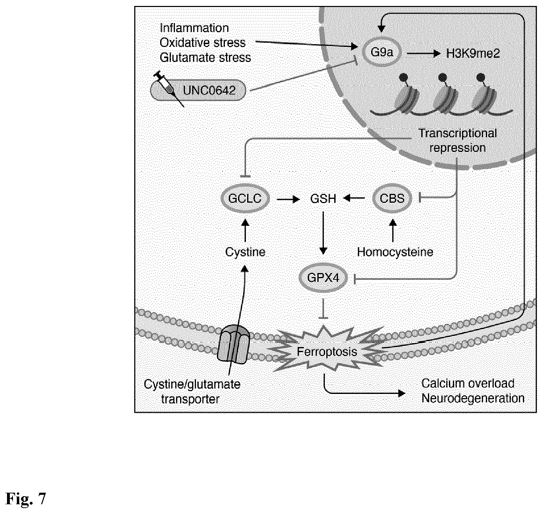

**Figure** 7 shows that G9a propagates neurodegeneration by enhancing ferroptosis. CNS inflammation enhances oxidative stress and increases extracellular glutamate levels, together inducing G9a activity in neurons. G9a catalyzes the repressive mark H3K9me2 that suppresses the expression of Gclc, Cbs and Gpx4. Thereby, G9a limits the availability of glutathione, induces lipid peroxidation and finally leads to ferroptosis, calcium overload and neuronal loss. Moreover, ferroptosis itself enhances G9a activity in a self-reinforcing feedback loop. Pharmacological inhibition of G9a by UNC0642 counteracts these effects and results in neuroprotection.

### EXAMPLES

The experimental study described herein aimed at identifying and characterizing an epigenetic modifier that is implicated in CNS inflammation. To this end, in vivo mouse studies, mouse and human neuronal cell culture assays and histological analysis of human specimens from MS patients and controls were conducted.

For mouse and human neuronal cell culture, experimental units correspond to individually prepared cultures derived from one pregnant mouse and individually differentiated batches of neuronal progenitor cells, respectively. Depending on the readout, up to five technical replicates were acquired per experimental unit. Technical replicates that deviated more than 20% from the mean of replicates, e.g., wells with dead cells, were excluded from the analysis.

For in vivo mouse studies, experimental units correspond to individual female mice. Age-matched mice were randomized into experimental groups before the start of the experiment and scored and analyzed with the investigator blinded to the treatment received. Mice that did not develop disease due to immunization failure were excluded from the analysis on the basis of predefined exclusion criteria.

For human histology, image analysis was conducted with the investigator blinded to subject identity and diagnosis. For all experiments, at least two independent experiments were carried out, sample sizes were calculated by the investigators on the basis of previous experience, endpoints and exclusion criteria were predefined. Data points identified as outliers by Grubbs' outlier test were excluded from downstream analysis.

The following materials and methods were used in the study:

### 1. Mice

All mice, C57BL/6J wild-type (The Jackson Laboratory), G9afl/fl (82) and G9afl/fl;Snap25-Cre (Snap25-Cre from the Jackson Laboratory) were kept under specific pathogen-free conditions in the central animal facility of the University Medical Center Hamburg-Eppendorf (UKE). Adult female mice in the age of 6-16 weeks were used.

### 2. Experimental autoimmune encephalomyelitis

Mice were immunized subcutaneously with 200 µg MOG35-55 peptide (Schafer-N) in CFA (Difco, cat. no. DF0639-60-6) containing 4 mg ml-1 Mycobacterium tuberculosis (Difco, cat. no. DF3114-33-8). In addition, 300 ng pertussis toxin (Calbiochem, cat. no. CAS70323-44-3) was injected intraperitoneally on the day of immunization and 48 hours later. Animals were scored daily for clinical signs by the following system: 0, no clinical deficits; 1, tail weakness; 2, hind limb paresis; 3, partial hind limb paralysis; 3.5, full hind limb paralysis; 4, full hind limb paralysis and fore limb paresis; 5, premorbid or dead. Animals reaching a clinical score ≥ 4 were euthanized according to the regulations of the local Animal Welfare Act. Where indicated, animals were injected intraperitoneally with 5 mg kg-1 UNC0642 (Sigma-Aldrich; cat. no. SML 1037) starting from day of disease onset. Littermate controls were used in all EAE experiments. UNC0642 was prediluted in DMSO and the final injection contained 10% DMSO ± UNC0642 and 90% Dulbecco's phosphate buffered saline (DPBS; Pan Biotech). The investigators were blind to the treatment in the EAE experiments.

### 3. Human induced pluripotent stem cell-derived neurons

Human iPSCs (ZIPi013-B) (83) were maintained under feeder-free conditions on Matrigel (Corning)-coated plates in mTeSR1 medium (Stem Cell Technologies, cat. no. 85850). For neuronal induction, iPSCs were dissociated with Accutase and seeded at a density of 3 × 10⁶ cells per well on AggreWell800 plates (10,000 cells per embryoid body (EB), Stem Cell Technologies) in SMADi Neural Induction Medium (SMADi NIM, Stem Cell Technologies, cat. no. 08582) supplemented with 10 Y-27632 (Stem Cell Technologies, cat. no. 72302). On day 6, EBs were harvested and cultivated on Matrigel-coated plates in SMADi NIM for 12 days. Newly formed neural rosettes were manually picked and cultured for another 4 days. In order to release neural precursor cells (NPCs), neural rosettes were dissociated with Accutase and were maintained for several passages at high density in Neural Progenitor Medium (Stem Cell Technologies, cat. no. 05833) on Matrigel-coated plates. HiPSC-derived NPCs were differentiated into neurons as previously described (84, 85) with some modifications. Briefly, NPCs were seeded at a density of 1 × 10⁵ cells per cm² in Neural Progenitor Medium onto Matrigel-coated plates. After 24 hours, the medium was replaced by neural differentiation medium (day 0 of differentiation) composed of Neurobasal Plus medium (Gibco, cat. no. A3582901) containing 1× B27 Plus Supplement (Gibco, cat. no. A3582801), 1×N2 Supplement-A (Stem Cell Technologies, cat. no. 07152), 1×MEM non-essential amino acids (Gibco, cat. no. 11140050), 1 µg ml⁻¹ laminin (Sigma-Aldrich, cat. no. L2020), 1 dibutyryl-cAMP (Stem Cell Technologies, cat. no. 73882), 10 ng ml⁻¹ L-ascorbic acid (Stem Cell Technologies, cat. no. 72132), 10 ng ml⁻¹ brain-derived neurotrophic factor (Stem Cell Technologies, cat. no. 78005) and 10 ng ml⁻¹ glial-derived neurotrophic factor (Stem Cell Technologies, cat. no. 78058). To promote a glutamatergic neuronal cell type, 5 cyclopamine (Stem Cell Technologies, cat. no. 72072) was additionally added to the medium during the first week of differentiation. On day 14, the cells were detached using Accutase and reseeded onto 96-well poly-L-ornithine/laminin-coated plates suited for confocal microscopy. Thereafter, cells were maintained in neural differentiation medium supplemented with CultureOne (Gibco, cat. no. A3320201) for up to 30 weeks to increase maturity.

### 4. Primary mouse neuronal cultures

For primary cortical cultures pregnant C57BL/6J, or G9afl/fl;Snap25-Cre mice were euthanized. To ensure comparability between genotypes, only embryos from heterozygous breeding's were used. Brainstem tissue of each embryo for genotyping was reserved and cortices were isolated, dissociated and cells were plated at a density of 1 × 10⁵ cells per 1 cm² on poly-D-lysine-coated wells (5 µM Sigma-Aldrich, cat. no. A-003-M). If not stated otherwise, cells were maintained in neurobasal plus medium (supplemented with B27 plus, penicillin, streptomycin and L-glutamine; Gibco, cat. no. A3582901) at 37°C, 5% CO² and a relative humidity of 98%, and treated with 1 µM cytarabine (Sigma-Aldrich, cat. no. BP383) at 1 day in vitro (div) to inhibit glial cell proliferation. If no cytarabine was applied cells were maintained in PNBM medium (supplemented with neural cell survival factor, gentamycin and L-glutamine; Lonza). Throughout, cultures after 14-23 div were used for experiments. G9a inhibitor UNC0642 was used in a concentration of 1 µM and cultures were pre-treated 24 hours before further experimental procedures.

### 5. Human immunohistochemistry

CNS tissues were fixed with 4% paraformaldehyde and embedded in paraffin. After deparaffination and antigen retrieval (Pascal Tris EDTA ph 9.0), the 2 µm sections were incubated with a peroxidase-blocking solution (Dako, cat. No. K0672) to inactivate endogenous peroxidases and unspecific binding was blocked (PBS + 10% goat serum). Tissue sections were incubated overnight with a mouse anti-H3K9me2 (Abcam, cat. no. ab1220, 1:200) and a rabbit anti-NeuN AlexaFluor 488 labelled (Abcam, cat. no. ab190195, 1:50). H3K9me2 bound antibodies were visualized using a donkey anti-mouse IgG2a (Jackson Immunoresearch, cat. no. 715-605-151, 1:200). Immunostained human samples were scanned using the Pannoramic 250 FLASH II (3DHISTECH) Digital Slide Scanner at 20× magnification.

### 6. RNAscope in situ hybridization

RNAscope Fluorescent Multiplex V2 (Advanced Cell Diagnostic (ADC), cat. no. 323100) was performed according to the manufacturer standard protocol for PFA fixed tissue. The human probe Hs-GPX4 (ACD, cat. no. 456851) and Hs-Snap25 (ACD, 518851) were commercially available from ACD. RNAscope human samples were scanned using the Pannoramic 250 FLASH II (3DHISTECH) Digital Slide Scanner at 20× magnification. GPX4+ SNAP25+ neurons were quantified by a blinded experimenter using a custom-made script, which was based on Cognition Network Language (Definiens Cognition Network Technology; Definiens Developer XD software).

### 7. Mouse immunohistochemistry

For histopathology of EAE mice, spinal cord tissue was fixed, decalcified, dehydrated, cast in paraffin and stained according to the standard procedures of the UKE Mouse Pathology Facility. The tissue was stained with hematoxylin (blue color) for orientation, followed by immunolabeling, that was visualized using the avidin-biotin complex technique with 3,3'-diaminobenzidine (brown stain). Slides were analyzed with a NanoZoomer 2.0-RS digital slide scanner and NDP.view2 software (Hamamatsu). CD3-positive cells were quantified in the white matter tract of the spinal cord using a customized counting mask with Fiji (ImageJ). NeuN-positive cells (neurons) were manually counted in the ventral horn outflow tract of the spinal cord. Area of 4-HNE and Iba1 stainings were analyzed with a customized mask using Fiji (ImageJ). Analysis conditions were standardized across all conditions. At least three images were analyzed per animal and the mean per animal was used for subsequent statistical comparisons.

### 8. Immunocytochemistry

Immunocytochemistry was performed as described previously (15). HiPSC-neurons and mouse neurons were cultivated on 12 mm diameter cover slips or on 96-well plates suited for confocal microscopy, fixed with 4% paraformaldehyde (PFA), incubated in 10% normal donkey's serum (NDS) containing 0.25% Triton X-100, and subsequently immunolabeling was performed. Immunostainings were analyzed with a Zeiss LSM 700 confocal microscope.

### 9. RealTime-Glo cell viability assay

RealTime-Glo (Promega, cat. no. G9711) MT cell viability substrate and NanoLuc Enzyme was mixed together, added to neuronal cultures and incubated for 5 hours for equilibration of luminescence signal before respective treatments were applied. Luminescence was acquired with a Spark 10M multimode microplate reader (Tecan) at 37°C and 5% CO₂ every 30 min over a total time period of 24 hours. At least five technical replicates per condition were used. For analysis every well's data point was normalized to its last value before the stressor was added and then normalized to the mean of the control wells for every time point using a customized MATLAB script. Thereby, it was normalized for well-to-well seeding variability.

### 10. Cell death induction

Primary neurons were subjected to dedicated compounds initiating either apoptosis (stauro-sporine), necroptosis (TNF-α + LCL-161 + Z-VAD-FMK, or shikonin), or ferroptosis (RSL3, or erastin) for 48 h in G9afl/fl;Snap25-Cre cultures, or cultures pre-treated for 24 hours with 1 µM UNC0642. Together with the cell death inducing compounds, CellToxTM Green Cytotoxicity Assay (Promega) was added to primary neurons 1:2000. Before fixation with 4% paraformaldehyde, cells were counterstained with Hoechst 3342. Neurons were imaged on a Zeiss LSM 700 confocal microscope using 20× magnification. Dyeing cells were quantified with Fiji (ImageJ) as displayed by bright green nuclear fluorescence and quantified as percentage of total cells, measured by the number of Hoechst positive nuclei.

### 11. Real-time PCR

RNA was extracted using RNeasy Mini Kit (Qiagen) and subsequently reverse-transcribed to complementary DNA with the RevertAid H Minus First Strand cDNA Synthesis Kit (Thermo Fisher Scientific) according to the manufacturer's instructions. Gene expression was analyzed by real-time PCR performed in an ABI Prism 7900 HT Fast Real-Time PCR System (Applied Biosystems) using TaqMan Gene Expression Assays (Thermo Fisher Scientific). Gene expression was calculated as 2-ΔCt relative to Tbp as the endogenous control.

### 12. Calcium imaging

Primary neuronal cultures were seeded on Ibidi 60 µ-Dish Quad (cat. No. 80411) plates. Cultures were infected with an AAV7 containing pAAV-Syn-GCamp6f-WPRE-SV40 (86) (Addgene 100837) at 7-10 div with a 20,000-fold multiplicity of infection (MOI). AAV particles were produced according to the standard procedures of the UKE vector facility. Images were acquired with a confocal LSM 700 laser scanning confocal microscope (Zeiss) every 0.48 seconds with a 20× magnification in an imaging chamber maintaining 37 °C and 5% CO₂. Infected cultures were imaged in the respective culture medium. Before applying the indicated chemicals, 5-10 min of baseline activity was first recorded. At the end of the recording, 8 µM ionomycin was applied to induce the maximum cellular calcium response that was used for normalization. For data analysis, mean fluorescence values of every cell were measured using Fiji software (NIH) and normalized to the maximal calcium response after ionomycin challenge. For each cell, mean AUC of the calcium response was calculated using a custom R script.

### 13. Oxidative stress detection

For the detection of oxidative stress in response to glutamate treatment, CellROX green reagent (Thermofisher) was used. Neuronal cultures were stimulated with 20 µM glutamate for a total incubation time of 1 hour. CellROX reagent was added to the wells in a 5 µM final concentration after 30 min of glutamate stress. Hoechst 33342, a cell permeant nuclear counterstain, was added for 10 min. Cells were washed two times with PBS before image acquisition. The cells were subsequently imaged on a Zeiss LSM 700 confocal microscope using 20× magnification. Immunofluorescence of nuclear CellROX dye was quantified using Fiji (ImageJ).

### 14. Glutathione measurement

GSH-Glo Glutathione assay (Promega) was used as described by the manufacturer. Primary cortical neurons were cultured in a 96-well plate and stimulated with glutamate for 2 hours. Cells were washed with PBS and GSH-Glo reagent was added to the wells for 30 min at room temperature, followed by the addition of luciferin detection reagent. After 15 min, luminescence was detected at a Spark 10M multimode microplate reader (Tecan).

### 15. Neuronal nuclei isolation and cell sorting

Nuclei of mouse spinal cords were isolated with the Nuclei Isolation Kit (Sigma Aldrich, cat. no. NUC101) according to the manufacturer's protocol with minor modifications. Briefly, mice were sacrificed with CO² and perfused with cold PBS. Whole spinal cords were removed and mechanically dissociated with a scalpel on a petri dish placed on a cooled metal block. Tissue was added to 2 ml of EZ buffer (Sigma Aldrich, cat. no. NUC101) and further dissociated using a glass douncer (Sigma-Aldrich D9063). After 5 min incubation on ice, homogenate was centrifuged (500 g, 5 min, 4 °C) and the pellet was washed in 2 ml of EZ buffer, followed by two washing steps in nuclei incubation buffer (340 mM sucrose, 2 mM MgCl2, 25 mM KCl, 65 mM glycerophosphate, 5% glycerol, 1 mM EDTA, 1% BSA). Nuclei were filtered using a 30 µM filter and directly stained with primary labelled NeuN antibody (ab 190565, Abcam) and propidium iodide. NeuN-positive nuclei were sorted using a BDAriaIII cell sorter (BD Bioscience). RNA for real-time PCR was processed as described above.

### 16. Neuronal nuclei isolation and flow cytometry

Nuclei of mouse spinal cords were isolated as described above. After filtering of the homogenate, nuclei were fixed with methanol for 10 min on ice, followed by two washing steps with nuclei incubation buffer. For intranuclear staining, nuclei were permeabilized with nuclei incubation buffer supplemented with 0.5% Triton-X-100 for 15 min on ice. After blocking with nucleus incubation buffer and 10% NDS, primary antibody was added for 1 hour at room temperature, followed by a washing step and subsequent incubation in secondary antibody for 30 min at room temperature. Immunofluorescence was acquired on an LSR II FACS analyzer (BD Biosciences). Data analysis was performed with the FlowJo v.10 analysis software (FlowJo LLC).

### 17. Isolation of CNS-infiltrating immune cells and flow cytometry

Mice were perfused intracardially with ice-cold PBS immediately after killing to remove blood from the intracranial vessels. The brain and spinal cord were prepared with sterile instruments, minced with a scalpel and incubated with agitation in Roswell Park Memorial Institute medium 1640 (PAA) containing 1 mg ml-1 collagenase A (Roche) and 0.1 mg ml⁻¹ DNase I recombinant, RNase-free (Roche) for 45 min at 37 °C. Tissue was triturated through a 100-µm cell strainer and washed with PBS (pellet was centrifuged at 300g for 10 min at 4 °C). The homogenized tissue was resuspended in 30% isotonic Percoll (GE Healthcare) and carefully underlaid with 78% isotonic Percoll. After gradient centrifugation (1,500g for 30 min at 4 °C), CNS-infiltrating immune cells were recovered from the gradient interphase and washed in ice-cold PBS. Single-cell suspensions were stained in the presence of 123 count eBeads (Thermofisher). Data was acquired on an LSR II FACS analyzer (BD Biosciences). Data analysis was performed with the FlowJo v.10 analysis software (FlowJo LLC).

### 18. Statistical analysis

Flow cytometric data were analyzed by using FlowJo (LLC). Images were analyzed using Fiji software (NIH). Experimental data obtained from Real-time Glo cell viability assay was analyzed with a customized MATLAB script and calcium imaging data were processed using a customized R script. Further experimental data were analyzed with the Prism 9 software (GraphPad Software) for Mac and are presented as the means ± s.e.m. Unless otherwise stated, differences between two experimental groups were determined using an unpaired, two-tailed Student's t-test; differences between three or more experimental groups were determined using one-way analysis of variance (ANOVA) with Tukey's post hoc test; differences between two or more experimental groups depending on two different variables were analyzed using two-way ANOVA with Tukey's post hoc test. Statistical analysis of the clinical scores in the EAE experiments was performed by applying a Mann-Whitney U-test to the cumulative score of each individual animal. Significant results are indicated by ^{∗}P < 0.05, ^{∗∗}P < 0.01 and ^{∗∗∗}P < 0.001.

### Example 1: Candidate gene identification

To identify epigenetic modifiers implicated in CNS inflammation, a neuronal translatome profiling data set of inflamed spinal cord motor neurons of EAE mice (13) was leveraged and ranked de-regulated candidates by statistical significance. The dataset focuses on known epigenetic regulators (dbEM - a Database of Epigenetic Modifiers) that were differentially expressed in inflamed versus healthy motor neurons. Differential expression analysis was performed with DESeq2 (v.1.14.1) calling genes with a minimal twofold change and false discovery rate (FDR)-adjusted P < 0.05 differentially expressed. It was found that histone methyltransferase G9a, encoded by the gene Ehmt2, was one of the epigenetic regulators that is differentially expressed in inflamed versus healthy motor neurons, as can be seen in Figure 1B. The induction of the Ehmt2 gene was validated by qPCR of sorted neuronal nuclei from inflamed spinal cords of EAE mice.

### Example 2: Detection of G9a activity in CNS inflammation

To assess the catalytic activity of G9a in the context of CNS inflammation, H3K9me2 levels in neuronal nuclei in EAE were measured in comparison to healthy spinal cord by flow cytometry. As can be seen in **Figure 1C****,** an increase of H3K9me2 levels in neuronal nuclei was found during acute (day 15) and chronic (day 30) EAE, but not prior to disease onset (day 11). This inflammation-dependent induction of H3K9me2 was accompanied by neuronal loss, as quantified by the percentage of NeuN-positive neuronal nuclei in spinal cords of acute and chronic EAE mice, as depicted in **Figure 1D**.

To assess the translatability of the findings in mice to human disease, the induction of H3K9me2 was investigated in MS brain tissue. As can be seen in **Figure 1D****,** a significant increase in H3K9me2 immunofluorescence signal in normal appearing grey matter (NAGM) as well as in cortical MS lesions was observed compared to control brain tissue. This is shown in **Figure 1E****.** This supports the conclusion that there is a widespread induction of G9a activity in MS brains.

To further explore whether H3K9me2 induction might be a general feature of stress-induced neurodegeneration, glutamate was applied to primary cortical cultures as a model of excitotoxicity during neuroinflammation (43, 44). Resembling the in vivo situation, glutamate exposure of primary cortical cultures led to an upregulation of H3K9me2, as shown in **Figure 1F**. The upregulation was associated with significantly decreased cell viability, as can be seen from **Figure 1G****.** Notably, stimulation with other inflammatory mediators such as tumor necrosis factor-alpha (TNF-α) or interferon-gamma (IFN-y) did not increase H3K9me2 levels (data not shown). Moreover, glutamate stress did not result in significant changes of other repressive (H3K9me1, H3K9me3, H3K27me3) or permissive (H3K27ac) epigenetic marks, nor did it change the levels of DNA-methyltransferase 3a (DNMT3a). Only H3K9me1, which is also catalyzed by G9a, showed a trend towards being induced by glutamate exposure.

To assess the upstream mechanism of H3K9me2 induction during glutamate toxicity, AP5 or EGTA were applied to block NMDA receptors or prevent calcium influx from the extracellular space, respectively. Both substances potently suppressed the induction of H3K9me2 in response to glutamate indicating NMDA receptor-mediated calcium influx from the extracellular space as an inducer of increased G9a activity. Besides H3K9me2 levels, NMDA receptor blockage by AP5 also rescued cellular survival. Together, both CNS inflammation in vivo and glutamate excitotoxicity in neuronal cultures resulted in increased G9a-dependent H3K9me2 levels and neuronal loss.

### Example 3: Impact of G9a and H3K9me2 on neuronal survival during excitotoxicity

To further explore the impact of G9a and H3K9me2 on neuronal survival during excitotoxicity two strategies for G9a perturbation were established, namely genetic disruption in G9a^{fl/fl}; Snap25-Cre neurons and pharmacological inhibition by the small molecule UNC0642 (45). Genetic disruption of G9a in primary cortical neuron cultures resulted in 90% reduction of Ehmt2 mRNA (data not shown), 76% reduction of G9a protein and 67% reduction of H3K9me2 signal, as shown in **Figure** 2A. Inhibition of the catalytic domain of G9a by UNC0642 did not change the G9a protein level but led to 45% reduction of H3K9me2 signal, as shown in **Figure 2B****.** Next, the potential of both perturbations to modulate glutamate-mediated neuronal cell death was investigated. As experimental readouts real time measurements of cell viability and toxic calcium overload in glutamate-stimulated primary neuronal cultures were employed. As set out in **Figure 2C and 2D****,** genetic disruption of G9a in G9a^{fl/fl}, Snap25-Cre neurons led to increased neuronal viability and reduced toxic calcium accumulation in comparison to G9a^{fl/fl} controls that was comparable to NMDA receptor blockage by AP5. To evaluate the potential of pharmacological G9a inhibition neuronal cultures were pre-treated for 24 hours with the G9a-specific small molecule inhibitor UNC0642 and obtained a similar rescue of cell viability and toxic calcium accumulation as seen for genetic G9a disruption. This is depicted in **Figure 2E-F****.** Moreover, G9a-deficient neurons from G9a^{fl/fl} Snap25-Cre mice were treated with UNC0642 and no additional rescue effect during glutamate stress was observed supporting a specific action of UNC0642 on G9a function. Together, G9a activity fostered toxic calcium overload and diminished neuronal survival after glutamate stress.

### Example 4: G9a potentiates oxidative stress in neurons

Since neuronal glutamate exposure as well as inflammatory challenges result in an excess of reactive oxygen species (8, 46), it was tested whether G9a activity might compromise neuronal resistance to oxidative stress. First, CellROX green was used to measure oxidative stress in primary neuronal cultures after glutamate exposure. While glutamate stimulation of G9a^{fl/fl} controls resulted in strong oxidative stress within one hour, genetic G9a disruption in G9a^{fl/fl};Snap25-Cre neurons led to a near-complete rescue to the level of cultures without glutamate treatment, as shown in **Figure 3A****.** Similarly, pre-treatment with UNC0642 reduced the generation of ROS close to baseline levels as seen without glutamate, as shown in **Figure 3B****.** To test G9a perturbation in a dedicated model with extensive oxidative stress, acute hydrogen peroxide stimulation was performed. It was found that both genetic G9a disruption in G9a^{fl/fl};Snap25-Cre neurons and G9a inhibition by UNC0642 rescued hydrogen peroxide-induced calcium overload in comparison to respective controls, as shown in **Figures 3C-D**. Notably, hydrogen peroxide treatment itself led to a pronounced upregulation of H3K9me2, indicating a vicious circle that further drives oxidative stress. Importantly, UNC0642 treatment was still able to overcome this effect. This shows that G9a exacerbates oxidative stress and consecutive calcium overload.

### Example 5: G9a induces neuronal cell death via ferroptosis

As G9a perturbation supported neuronal viability, a downstream effect of G9a in a central pathway of programmed cell death was hypothesized. Therefore, dedicated compounds were used to induce different forms of programmed cell death that have been implicated in neurodegeneration and oxidative stress and assessed their dependence on G9a function. While neither apoptosis nor necroptosis was affected by G9a perturbation, ferroptosis induced by either erastin or RSL3 was substantially rescued in G9a knockout neurons and after G9a inhibition with UNC0642, as can be seen in **Figures 4A-B****.** Moreover, G9a activity was measured by quantification of H3K9me2 immunofluorescence in response to the activation of the different cell death pathways. Again, no changes in H3K9me2 signal in apoptosis or necroptosis were observed. However, ferroptosis induction via erastin or RSL3 resulted itself in a significant upregulation of H3K9me2, as shown in **Figure 4C****.** Similar to the finding after hydrogen peroxide treatment, this suggests that G9a participates in a self-reinforcing feedback loop, where G9a-dependent H3K9me2 supports ferroptosis and ferroptosis drives H3K9me2. Importantly, upregulation of H3K9me2 in response to ferroptosis induction could be effectively prevented by UNC0642.

To further elucidate the involvement of ferroptosis in glutamate-induced cell death, cyclooxygenase-2 (COX-2) was chosen as a widely used biomarker of ferroptosis (47). Indeed, glutamate excitotoxicity led to a markedly higher percentage of COX-2-positive cells, whereas pharmacological G9a inhibition almost completely abolished its induction, as shown in **Figure 4D**. As another way to explore the involvement of ferroptosis in glutamate excitotoxicity the potential of ferroptosis inhibitors to rescue glutamate-induced calcium overload was investigated. Besides the antioxidant ferrostatin-1 also the iron chelator alpha-difluoro-methylornithine (DFMO), and the lipophilic radical-trapping antioxidant coenzyme Q10 rescued glutamate-induced calcium overload, as shown in **Figure 4E**. This substantiates ferroptosis as a central cell death pathway in glutamate excitotoxicity. Moreover, pre-treatment with the G9a inhibitor UNC0642 did not further potentiate the rescue effects of ferroptosis inhibition suggesting a shared pathway of both interventions, as shown in **Figure 4E**.
Finally, it was assessed whether G9a perturbation could increase the levels of glutathione, a key anti-ferroptotic effector molecule with antioxidant function. Indeed, while glutamate treatment reduced the levels of glutathione in primary neuronal cultures, both genetic G9a disruption in G9a^{fl/fl};Snap25-Cre neurons as well as pharmacological G9a inhibition by UNC0642 led to a partial rescue of glutathione levels, as shown in **Figures 4F-G****.** In summary, these findings suggest a pro-ferroptotic function of G9a that can be counteracted by pharmacological inhibition with UNC0642.

### Example 6: G9a amplifies neurodegeneration and represses anti-ferroptotic genes

To explore the potential of pharmacological G9a inhibition as a neuroprotective treatment strategy in vivo, EAE animals were injected once daily with 5 mg kg⁻¹ body weight UNC0642 or vehicle control, starting at symptom onset. In accordance with the in vitro findings, treatment with UNC0642 resulted in a significantly ameliorated EAE disease course compared to vehicle-treated mice, which was particularly prominent in the chronic phase of EAE. This is shown in **Figures 5A-B****.** UNC0642 treatment further led to reduced neuronal loss in the spinal cord ventral horn of chronic EAE mice, as shown in **Figure 5C**. At the same time, immune cell infiltration, microglia activation and body weight changes were not altered, as can be seen in **Figure 5D****.**

Next, the effect of UNC0642 in vivo treatment on epigenetic silencing via H3K9me2 and the induction of ferroptosis was investigated. The applied UNC0642 treatment regime inhibited H3K9me2 induction and led to a complete normalization of H3K9me2 levels in neuronal nuclei of EAE animals, as shown in **Figure 5E****.** Moreover, as set out in **Figure 5F****,** increased levels of the histopathological ferroptosis marker 4-hydroxy-2-nonenal (4-HNE) were found in EAE, which was significantly reduced by UNC0642 treatment, as shown in **Figure 5G****.** It was hypothesized that this effect might be driven by derepression of anti-ferroptotic genes. To test this hypothesis, NeuN-positive neuronal nuclei were sorted from the spinal cords of healthy controls, EAE mice and EAE mice treated with UNC0642. mRNA expression of ferroptosis-related genes was assessed by qPCR. Notably, it was observed that many of the genes with anti-ferroptotic function showed a transcriptional suppression during EAE, as can be taken from **Figure 5H****.** Among them Gpx4, encoding the primary cellular defense system against ferroptosis by converting lipid peroxides into non-toxic lipid alcohols (47) and Gclc, encoding the glutamate-cysteine ligase catalytic subunit, the first rate limiting enzyme of glutathione synthesis (48), as depicted in **Figure 5I****.** Strikingly, UNC0642 treatment in EAE significantly boosted the expression of Gpx4 and Gclc as well as Cbs, encoding cystathionine β-synthase, the first enzyme of the transsulfuration pathway, an alternative way to fuel glutathione production (49), and Nfe212, encoding nuclear factor erythroid 2-related factor 2, a transcription factor that drives several anti-oxidant genes including Gclc (50-52). Moreover, multiple glutathione S-transferases followed the same pattern, showing suppression in EAE and derepression upon UNC0642 treatment.

Together, these findings exemplify a strong impact of G9a-mediated transcriptional regulation on the glutathione pathway. In contrast, ferroptosis suppressor protein 1 (Fsp1), a glutathione independent anti-ferroptotic molecule (53) showed no significant regulation (data not shown). It was concluded that G9a activity led to transcriptional repression of anti-ferroptotic genes, many of which are implicated in the generation of the intracellular antioxidant glutathione. In line with these findings, a potent neuroprotective effect of pharmacological G9a inhibition manifesting in increased neuronal survival and improved clinical outcome in CNS inflammation was observed.

### Example 7: Anti-ferroptotic GPX4 is suppressed in MS and induced by G9a inhibition

To translate the above findings, the expression level of the main regulator of ferroptosis, GPX4, was investigated in human brain slices of MS patients by RNAScope in situ hybridization. As shown in **Figure 6A****,** a reduction of GPX4+ dots per neuron in NAGM and cortical lesions in brain sections of MS patients was found, reflecting the transcriptional repression of Gpx4 in the EAE mouse model. Further, a prominent increase of H3K9me2 levels were detected in response to erastin-induced ferroptosis in neuronal cultures derived from human induced pluripotent stem cells (hiPSC), which was rescued by G9a inhibition with UNC0642, as shown in **Figure 6B****.** This again reflected the observations in murine neuronal cultures and corroborated induction of G9a activity by ferroptosis and potent suppression by UNC0642 in human cells. Next, the transcriptional regulation of ferroptosis-related genes by G9a was investigated. In line with the findings in UNC0642-treated EAE mice, a significant upregulation of GPX4 and GCLC was found in response to G9a inhibition via UNC0642 in erastin-treated human neurons, as shown in **Figure 6C****.** Of note, also the expression of SLC7A11, coding for the cystine/glutamate transporter that provides the building blocks for glutathione synthesis, was highly upregulated by G9a inhibition. This is also shown in **Figure 6C****.**

In summary, these results showed an induction of H3K9me2 and repression of GPX4 in neurons of MS patients. Both effects could be reversed by pharmacological G9a inhibition in human neuronal cultures. Together with the evidence from EAE and murine neuronal cell culture, the above findings indicate a deleterious induction of neuronal G9a activity during neuroinflammation, as shown in **Figure 7****.** The resulting increase of repressive H3K9me2 leads to epigenetic silencing of key anti-ferroptotic genes including Gpx4, Gclc and Cbs, causing a depletion of glutathione, accumulation of reactive oxygen species and ultimately ferroptotic cell death. Epigenetic reset of neurons by G9a inhibition counteracts this maladaptive cascade and improves neuronal survival and clinical outcome.

### REFERENCES

1. M. Prinz, T. Masuda, M. A. Wheeler, F. J. Quintana, Microglia and Central Nervous System-Associated Macrophages-From Origin to Disease Modulation. Annu Rev Immunol 39, 251-277 (2021).
2. M. A. Friese, B. Schattling, L. Fugger, Mechanisms of neurodegeneration and axonal dysfunction in multiple sclerosis. Nat Rev Neurol 10, 225-238 (2014).
3. C. A. Dendrou, L. Fugger, M. A. Friese, Immunopathology of multiple sclerosis. Nat Rev Immunol 15, 545-558 (2015).
4. S. Luchetti, N. L. Fransen, C. G. van Eden, V. Ramaglia, M. Mason, I. Huitinga, Progressive multiple sclerosis patients show substantial lesion activity that correlates with clinical disease severity and sex: a retrospective autopsy cohort analysis. Acta Neuropathol 135, 511-528 (2018).
5. M. Absinta, H. Lassmann, B. D. Trapp, Mechanisms underlying progression in multiple sclerosis. Curr Opin Neurol 33, 277-285 (2020).
6. R. M. Ransohoff, How neuroinflammation contributes to neurodegeneration. Science 353, (2016).
7. F. Dangond, A. Donnelly, R. Hohlfeld, C. Lubetzki, S. Kohlhaas, L. Leocani, O. Ciccarelli, B. Stankoff, M. P. Sormani, J. Chataway, F. Bozzoli, F. Cucca, L. Melton, T. Coetzee, M. Salvetti, Facing the urgency of therapies for progressive MS - a Progressive MS Alliance proposal. Nat Rev Neurol 17, 185-192 (2021).
8. L. Haider, M. T. Fischer, J. M. Frischer, J. Bauer, R. Hoftberger, G. Botond, H. Esterbauer, C. J. Binder, J. L. Witztum, H. Lassmann, Oxidative damage in multiple sclerosis lesions. Brain 134, 1914-1924 (2011).
9. G. R. Campbell, I. Ziabreva, A. K. Reeve, K. J. Krishnan, R. Reynolds, O. Howell, H. Lassmann, D. M. Turnbull, D. J. Mahad, Mitochondrial DNA deletions and neurodegeneration in multiple sclerosis. Ann Neurol 69, 481-492 (2011).
10. R. Macrez, P. K. Stys, D. Vivien, S. A. Lipton, F. Docagne, Mechanisms of glutamate toxicity in multiple sclerosis: biomarker and therapeutic opportunities. The Lancet Neurology 15, 1089-1102 (2016).
11. L. Schirmer, D. Velmeshev, S. Holmqvist, M. Kaufmann, S. Werneburg, D. Jung, S. Vistnes, J. H. Stockley, A. Young, M. Steindel, B. Tung, N. Goyal, A. Bhaduri, S. Mayer, J. B. Engler, O. A. Bayraktar, R. J. M. Franklin, M. Haeussler, R. Reynolds, D. P. Schafer, M. A. Friese, L. R. Shiow, A. R. Kriegstein, D. H. Rowitch, Neuronal vulnerability and multilineage diversity in multiple sclerosis. Nature 573, 75-82 (2019).
12. S. Faissner, J. R. Plemel, R. Gold, V. W. Yong, Progressive multiple sclerosis: from pathophysiology to therapeutic strategies. Nat Rev Drug Discov 18, 905-922 (2019).
13. B. Schattling, J. B. Engler, C. Volkmann, N. Rothammer, M. S. Woo, M. Petersen, I. Winkler, M. Kaufmann, S. C. Rosenkranz, A. Fejtova, U. Thomas, A. Bose, S. Bauer, S. Träger, K. K. Miller, W. Brück, K. E. Duncan, G. Salinas, P. Soba, E. D. Gundelfinger, D. Merkler, M. A. Friese, Bassoon proteinopathy drives neurodegeneration in multiple sclerosis. Nat Neurosci 22, 887-896 (2019).
14. S. C. Rosenkranz, A. A. Shaposhnykov, S. Träger, J. B. Engler, M. E. Witte, V. Roth, V. Vieira, N. Paauw, S. Bauer, C. Schwencke-Westphal, C. Schubert, L. C. Bal, B. Schattling, O. Pless, J. van Horssen, M. Freichel, M. A. Friese, Enhancing mitochondrial activity in neurons protects against neurodegeneration in a mouse model of multiple sclerosis. eLife 10, (2021).
15. B. Schattling, K. Steinbach, E. Thies, M. Kruse, A. Menigoz, F. Ufer, V. Flockerzi, W. Bruck, O. Pongs, R. Vennekens, M. Kneussel, M. Freichel, D. Merkler, M. A. Friese, TRPM4 cation channel mediates axonal and neuronal degeneration in experimental autoimmune encephalomyelitis and multiple sclerosis. Nat Med 18, 1805-1811 (2012).
16. M. A. Friese, M. J. Craner, R. Etzensperger, S. Vergo, J. A. Wemmie, M. J. Welsh, A. Vincent, L. Fugger, Acid-sensing ion channel-1 contributes to axonal degeneration in autoimmune inflammation of the central nervous system. Nat Med 13, 1483-1489 (2007).
17. J. Yan, C. P. Bengtson, B. Buchthal, A. M. Hagenston, H. Bading, Coupling of NMDA receptors and TRPM4 guides discovery of unconventional neuroprotectants. Science 370, (2020).
18. J. A. Black, S. Liu, B. C. Hains, C. Y. Saab, S. G. Waxman, Long-term protection of central axons with phenytoin in monophasic and chronic-relapsing EAE. Brain 129, 3196-3208 (2006).
19. M. S. Woo, F. Ufer, N. Rothammer, G. Di Liberto, L. Binkle, U. Haferkamp, J. K. Sonner, J. B. Engler, S. Hornig, S. Bauer, I. Wagner, K. Egervari, J. Raber, R. M. Duvoisin, O. Pless, D. Merkler, M. A. Friese, Neuronal metabotropic glutamate receptor 8 protects against neurodegeneration in CNS inflammation. J Exp Med 218, (2021).
20. Y. Gilgun-Sherki, H. Panet, E. Melamed, D. Offen, Riluzole suppresses experimental autoimmune encephalomyelitis: implications for the treatment of multiple sclerosis. Brain Research 989, 196-204 (2003).
21. T. Smith, A. Groom, B. Zhu, L. Turski, Autoimmune encephalomyelitis ameliorated by AMPA antagonists. Nature Medicine 6, 62-66 (2000).
22. B. J. Andreone, M. Larhammar, J. W. Lewcock, Cell Death and Neurodegeneration. Cold Spring Harb Perspect Biol 12, (2020).
23. M. M. Farley, T. A. Watkins, Intrinsic Neuronal Stress Response Pathways in Injury and Disease. Annual Review of Pathology: Mechanisms of Disease 13, 93-116 (2018).
24. J. Y. Hwang, K. A. Aromolaran, R. S. Zukin, The emerging field of epigenetics in neurodegeneration and neuroprotection. Nat Rev Neurosci 18, 347-361 (2017).
25. K. P. Bhat, H. Umit Kaniskan, J. Jin, O. Gozani, Epigenetics and beyond: targeting writers of protein lysine methylation to treat disease. Nat Rev Drug Discov, (2021).
26. H. U. Klein, C. McCabe, E. Gjoneska, S. E. Sullivan, B. J. Kaskow, A. Tang, R. V. Smith, J. Xu, A. R. Pfenning, B. E. Bernstein, A. Meissner, J. A. Schneider, S. Mostafavi, L. H. Tsai, T. L. Young-Pearse, D. A. Bennett, P. L. De Jager, Epigenome-wide study uncovers large-scale changes in histone acetylation driven by tau pathology in aging and Alzheimer's human brains. Nat Neurosci 22, 37-46 (2019).
27. L. Kular, M. Needhamsen, M. Z. Adzemovic, T. Kramarova, D. Gomez-Cabrero, E. Ewing, E. Piket, J. Tegner, S. Beck, F. Piehl, L. Brundin, M. Jagodic, Neuronal methylome reveals CREB-associated neuro-axonal impairment in multiple sclerosis. Clin Epigenetics 11, 86 (2019).
28. J. L. Huynh, P. Garg, T. H. Thin, S. Yoo, R. Dutta, B. D. Trapp, V. Haroutunian, J. Zhu, M. J. Donovan, A. J. Sharp, P. Casaccia, Epigenome-wide differences in pathology-free regions of multiple sclerosis-affected brains. Nat Neurosci 17, 121-130 (2014).
29. J. L. Huynh, P. Casaccia, Epigenetic mechanisms in multiple sclerosis: implications for pathogenesis and treatment. The Lancet Neurology 12, 195-206 (2013).
30. R. Meyer, R. Weissert, R. Diem, M. K. Storch, K. L. de Graaf, B. Kramer, M. Bähr, Acute Neuronal Apoptosis in a Rat Model of Multiple Sclerosis. The Journal of Neuroscience 21(16), 6214-6220 (2001).
31. D. Ofengeim, Y. Ito, A. Najafov, Y. Zhang, B. Shan, J. P. DeWitt, J. Ye, X. Zhang, A. Chang, H. Vakifahmetoglu-Norberg, J. Geng, B. Py, W. Zhou, P. Amin, J. Berlink Lima, C. Qi, Q. Yu, B. Trapp, J. Yuan, Activation of necroptosis in multiple sclerosis. Cell Rep 10, 1836-1849 (2015).
32. C. Picon, A. Jayaraman, R. James, C. Beck, P. Gallego, M. E. Witte, J. van Horssen, N. D. Mazarakis, R. Reynolds, Neuron-specific activation of necroptosis signaling in multiple sclerosis cortical grey matter. Acta Neuropathol 141, 585-604 (2021).
33. S. J. Dixon, K. M. Lemberg, M. R. Lamprecht, R. Skouta, E. M. Zaitsev, C. E. Gleason, D. N. Patel, A. J. Bauer, A. M. Cantley, W. S. Yang, B. Morrison, 3rd, B. R. Stockwell, Ferroptosis: an iron-dependent form of nonapoptotic cell death. Cell 149, 1060-1072 (2012).
34. X. Jiang, B. R. Stockwell, M. Conrad, Ferroptosis: mechanisms, biology and role in disease. Nat Rev Mol Cell Biol, (2021).
35. S. J. Dixon, B. R. Stockwell, The Hallmarks of Ferroptosis. Annual Review of Cancer Biology 3, 35-54 (2019).
36. H. Lassmann, J. van Horssen, Oxidative stress and its impact on neurons and glia in multiple sclerosis lesions. Biochim Biophys Acta 1862, 506-510 (2016).
37. M. T. Fischer, I. Wimmer, R. Hoftberger, S. Gerlach, L. Haider, T. Zrzavy, S. Hametner, D. Mahad, C. J. Binder, M. Krumbholz, J. Bauer, M. Bradl, H. Lassmann, Disease-specific molecular events in cortical multiple sclerosis lesions. Brain 136, 1799-1815 (2013).
38. S. Hametner, I. Wimmer, L. Haider, S. Pfeifenbring, W. Bruck, H. Lassmann, Iron and neurodegeneration in the multiple sclerosis brain. Ann Neurol 74, 848-861 (2013).
39. C. L. Hu, M. Nydes, K. L. Shanley, I. E. Morales Pantoja, T. A. Howard, O. A. Bizzozero, Reduced expression of the ferroptosis inhibitor glutathione peroxidase-4 in multiple sclerosis and experimental autoimmune encephalomyelitis. J Neurochem 148, 426-439 (2019).
40. C. Mukherjee, T. Kling, B. Russo, K. Miebach, E. Kess, M. Schifferer, L. D. Pedro, U. Weikert, M. K. Fard, N. Kannaiyan, M. Rossner, M. L. Aicher, S. Goebbels, K. A. Nave, E. M. Kramer-Albers, A. Schneider, M. Simons, Oligodendrocytes Provide Antioxidant Defense Function for Neurons by Secreting Ferritin Heavy Chain. Cell Metab 32, 259-272 e210 (2020).
41. Z. J. Wang, P. Zhong, K. Ma, J. S. Seo, F. Yang, Z. Hu, F. Zhang, L. Lin, J. Wang, T. Liu, E. Matas, P. Greengard, Z. Yan, Amelioration of autism-like social deficits by targeting histone methyltransferases EHMT1/2 in Shank3-deficient mice. Mol Psychiatry 25, 2517-2533 (2020).
42. Y. Zheng, A. Liu, Z. J. Wang, Q. Cao, W. Wang, L. Lin, K. Ma, F. Zhang, J. Wei, E. Matas, J. Cheng, G. J. Chen, X. Wang, Z. Yan, Inhibition of EHMT1/2 rescues synaptic and cognitive functions for Alzheimer's disease. Brain 142, 787-807 (2019).
43. D. Pitt, P. Werner, C. Raine, Glutamate excitotoxicity in a model of multiple sclerosis. Nature Medicine 6, 67-70 (2000).
44. K. Birkner, B. Wasser, T. Ruck, C. Thalman, D. Luchtman, K. Pape, S. Schmaul, L. Bitar, E. M. Kramer-Albers, A. Stroh, S. G. Meuth, F. Zipp, S. Bittner, betal-Integrin- and KV1.3 channel-dependent signaling stimulates glutamate release from Th17 cells. J Clin Invest 130, 715-732 (2020).
45. F. Liu, D. Barsyte-Lovejoy, F. Li, Y. Xiong, V. Korboukh, X. P. Huang, A. Allali-Hassani, W. P. Janzen, B. L. Roth, S. V. Frye, C. H. Arrowsmith, P. J. Brown, M. Vedadi, J. Jin, Discovery of an in vivo chemical probe of the lysine methyltransferases G9a and GLP. J Med Chem 56, 8931-8942 (2013).
46. O. Vergun, A. I. Sobolevsky, M. V. Yelshansky, J. Keelan, B. I. Khodorov, M. R. Duchen, Exploration of the role of reactive oxygen species in glutamate neurotoxicity in rat hippocampal neurones in culture. Journal of Physiology 531, 147-163 (2001).
47. W. S. Yang, R. SriRamaratnam, M. E. Welsch, K. Shimada, R. Skouta, V. S. Viswanathan, J. H. Cheah, P. A. Clemons, A. F. Shamji, C. B. Clish, L. M. Brown, A. W. Girotti, V. W. Cornish, S. L. Schreiber, B. R. Stockwell, Regulation of ferroptotic cancer cell death by GPX4. Cell 156, 317-331 (2014).
48. S. C. Lu, Regulation of glutathione synthesis. Mol Aspects Med 30, 42-59 (2009).
49. M. Eugene, M. R. Cranford, R. Banerjee, The Quantitatively Important Relationship between Homocysteine Metabolism and Glutathione Synthesis by the Transsulfuration Pathway and Its Regulation by Redox Changes. Biochemistry 39, 13005-13011 (2000).
50. K. F. Bell, J. H. Fowler, B. Al-Mubarak, K. Horsburgh, G. E. Hardingham, Activation of Nrf2-regulated glutathione pathway genes by ischemic preconditioning. Oxid Med Cell Longev 2011, 689524 (2011).
51. C. J. Harvey, R. K. Thimmulappa, A. Singh, D. J. Blake, G. Ling, N. Wakabayashi, J. Fujii, A. Myers, S. Biswal, Nrf2-regulated glutathione recycling independent of biosynthesis is critical for cell survival during oxidative stress. Free Radic Biol Med 46, 443-453 (2009).
52. C. Tonelli, I. I. C. Chio, D. A. Tuveson, Transcriptional Regulation by Nrf2. Antioxid Redox Signal 29, 1727-1745 (2018).
53. S. Doll, F. P. Freitas, R. Shah, M. Aldrovandi, M. C. da Silva, I. Ingold, A. Goya Grocin, T. N. Xavier da Silva, E. Panzilius, C. H. Scheel, A. Mourao, K. Buday, M. Sato, J. Wanninger, T. Vignane, V. Mohana, M. Rehberg, A. Flatley, A. Schepers, A. Kurz, D. White, M. Sauer, M. Sattler, E. W. Tate, W. Schmitz, A. Schulze, V. O'Donnell, B. Proneth, G. M. Popowicz, D. A. Pratt, J. P. F. Angeli, M. Conrad, FSP1 is a glutathione-independent ferroptosis suppressor. Nature 575, 693-698 (2019).
54. P. M. Matthews, Chronic inflammation in multiple sclerosis - seeing what was always there. Nat Rev Neurol 15, 582-593 (2019).
55. R. S. Klein, C. Garber, K. E. Funk, H. Salimi, A. K. Soung, M. Manivasagam, S., S. Agner, M. Cain, Neuroinflammation During RNA Viral Infections. Annual Review Immunology 37, 73-95 (2019).
56. M. R. Nichols, M. K. St-Pierre, A. C. Wendeln, N. J. Makoni, L. K. Gouwens, E. C. Garrad, M. Sohrabi, J. J. Neher, M. E. Tremblay, C. K. Combs, Inflammatory mechanisms in neurodegeneration. J Neurochem 149, 562-581 (2019).
57. D. Sulzer, R. N. Alcalay, F. Garretti, L. Cote, E. Kanter, J. Agin-Liebes, C. Liong, C. McMurtrey, W. H. Hildebrand, X. Mao, V. L. Dawson, T. M. Dawson, C. Oseroff, J. Pham, J. Sidney, M. B. Dillon, C. Carpenter, D. Weiskopf, E. Phillips, S. Mallal, B. Peters, A. Frazier, C. S. Lindestam Arlehamn, A. Sette, T cells from patients with Parkinson's disease recognize alpha-synuclein peptides. Nature 546, 656-661 (2017).
58. S. M. Matta, E. L. Hill-Yardin, P. J. Crack, The influence of neuroinflammation in Autism Spectrum Disorder. Brain Behav Immun 79, 75-90 (2019).
59. L. Gan, M. R. Cookson, L. Petrucelli, A. R. La Spada, Converging pathways in neurodegeneration, from genetics to mechanisms. Nat Neurosci 21, 1300-1309 (2018).
60. M. Kaufmann, H. Evans, A. L. Schaupp, J. B. Engler, G. Kaur, A. Willing, N. Kursawe, C. Schubert, K. E. Attfield, L. Fugger, M. A. Friese, Identifying CNS-colonizing T cells as potential therapeutic targets to prevent progression of multiple sclerosis. Med (N Y) 2, 296-312 e298 (2021).
61. B. A. C. Cree, J. A. Hollenbach, R. Bove, G. Kirkish, S. Sacco, E. Caverzasi, A. Bischof, T. Gundel, A. H. Zhu, N. Papinutto, W. A. Stern, C. Bevan, A. Romeo, D. S. Goodin, J. M. Gelfand, J. Graves, A. J. Green, M. R. Wilson, S. S. Zamvil, C. Zhao, R. Gomez, N. R. Ragan, G. Q. Rush, P. Barba, A. Santaniello, S. E. Baranzini, J. R. Oksenberg, R. G. Henry, S. L. Hauser, Silent progression in disease activity-free relapsing multiple sclerosis. Ann Neurol 85, 653-666 (2019).
62. M. A. Wheeler, I. C. Clark, E. C. Tjon, Z. Li, S. E. J. Zandee, C. P. Couturier, B. R. Watson, G. Scalisi, S. Alkwai, V. Rothhammer, A. Rotem, J. A. Heyman, S. Thaploo, L. M. Sanmarco, J. Ragoussis, D. A. Weitz, K. Petrecca, J. R. Moffitt, B. Becher, J. P. Antel, A. Prat, F. J. Quintana, MAFG-driven astrocytes promote CNS inflammation. Nature 578, 593-599 (2020).
63. H. He, Z. Hu, H. Xiao, F. Zhou, B. Yang, The tale of histone modifications and its role in multiple sclerosis. Hum Genomics 12, 31 (2018).
64. R. Alcala-Vida, J. Seguin, C. Lotz, A. M. Molitor, I. Irastorza-Azcarate, A. Awada, N. Karasu, A. Bombardier, B. Cosquer, J. L. G. Skarmeta, J. C. Cassel, A. L. Boutillier, T. Sexton, K. Merienne, Age-related and disease locus-specific mechanisms contribute to early remodelling of chromatin structure in Huntington's disease mice. Nat Commun 12, 364 (2021).
65. R. Nativio, G. Donahue, A. Berson, Y. Lan, A. Amlie-Wolf, F. Tuzer, J. B. Toledo, S. J. Gosai, B. D. Gregory, C. Torres, J. Q. Trojanowski, L. S. Wang, F. B. Johnson, N. M. Bonini, S. L. Berger, Dysregulation of the epigenetic landscape of normal aging in Alzheimer's disease. Nat Neurosci 21, 497-505 (2018).
66. N. Tsankova, W. Renthal, A. Kumar, E. J. Nestler, Epigenetic regulation in psychiatric disorders. Nat Rev Neurosci 8, 355-367 (2007).
67. D. G. Brickner, I. Cajigas, Y. Fondufe-Mittendorf, S. Ahmed, P. C. Lee, J. Widom, J. H. Brickner, H2A.Z-mediated localization of genes at the nuclear periphery confers epigenetic memory of previous transcriptional state. PLoS Biol 5, e81 (2007).
68. Z. Zhang, R. Zhang, Epigenetics in autoimmune diseases: Pathogenesis and prospects for therapy. Autoimmun Rev 14, 854-863 (2015).
69. R. Plummer, L. Vidal, M. Griffin, M. Lesley, J. de Bono, S. Coulthard, J. Sludden, L. L. Siu, E. X. Chen, A. M. Oza, G. K. Reid, A. R. McLeod, J. M. Besterman, C. Lee, I. Judson, H. Calvert, A. V. Boddy, Phase I study of MG98, an oligonucleotide antisense inhibitor of human DNA methyltransferase 1, given as a 7-day infusion in patients with advanced solid tumors. Clin Cancer Res 15, 3177-3183 (2009).
70. C. Segovia, E. San Jose-Eneriz, E. Munera-Maravilla, M. Martinez-Fernandez, L. Garate, E. Miranda, A. Vilas-Zornoza, I. Lodewijk, C. Rubio, C. Segrelles, L. V. Valcarcel, O. Rabal, N. Casares, A. Bernardini, C. Suarez-Cabrera, F. F. Lopez-Calderon, P. Fortes, J. A. Casado, M. Duenas, F. Villacampa, J. J. Lasarte, F. Guerrero-Ramos, G. de Velasco, J. Oyarzabal, D. Castellano, X. Agirre, F. Prosper, J. M. Paramio, Inhibition of a G9a/DNMT network triggers immune-mediated bladder cancer regression. Nat Med 25, 1073-1081 (2019).
71. A. Insinga, S. Monestiroli, S. Ronzoni, V. Gelmetti, F. Marchesi, A. Viale, L. Altucci, C. Nervi, S. Minucci, P. G. Pelicci, Inhibitors of histone deacetylases induce tumor-selective apoptosis through activation of the death receptor pathway. Nat Med 11, 71-76 (2005).
72. Y. Cheng, C. He, M. Wang, X. Ma, F. Mo, S. Yang, J. Han, X. Wei, Targeting epigenetic regulators for cancer therapy: mechanisms and advances in clinical trials. Signal Transduct Target Ther 4, 62 (2019).
73. R. Gupta, P. Saha, T. Sen, N. Sen, An augmentation in histone dimethylation at lysine nine residues elicits vision impairment following traumatic brain injury. Free Radic Biol Med 134, 630-643 (2019).
74. H. P. Mohammad, O. Barbash, C. L. Creasy, Targeting epigenetic modifications in cancer therapy: erasing the roadmap to cancer. Nat Med 25, 403-418 (2019).
75. S. Jan, M. I. Dar, R. Wani, J. Sandey, I. Mushtaq, S. Lateef, S. H. Syed, Targeting EHMT2/ G9a for cancer therapy: Progress and perspective. Eur J Pharmacol 893, 173827 (2021).
76. M. Conrad, V. E. Kagan, H. Bayir, G. C. Pagnussat, B. Head, M. G. Traber, B. R. Stockwell, Regulation of lipid peroxidation and ferroptosis in diverse species. Genes Dev 32, 602-619 (2018).
77. I. Alim, J. T. Caulfield, Y. Chen, V. Swarup, D. H. Geschwind, E. Ivanova, J. Seravalli, Y. Ai, L. H. Sansing, E. J. Ste Marie, R. J. Hondal, S. Mukherjee, J. W. Cave, B. T. Sagdullaev, S. S. Karuppagounder, R. R. Ratan, Selenium Drives a Transcriptional Adaptive Program to Block Ferroptosis and Treat Stroke. Cell 177, 1262-1279 e1225 (2019).
78. B. S. Xie, Y. Q. Wang, Y. Lin, Q. Mao, J. F. Feng, G. Y. Gao, J. Y. Jiang, Inhibition of ferroptosis attenuates tissue damage and improves long-term outcomes after traumatic brain injury in mice. CNS Neurosci Ther 25, 465-475 (2019).
79. D. Tang, X. Chen, R. Kang, G. Kroemer, Ferroptosis: molecular mechanisms and health implications. Cell Res 31, 107-125 (2021).
80. A. Anandhan, M. Dodson, C. J. Schmidlin, P. Liu, D. D. Zhang, Breakdown of an Ironclad Defense System: The Critical Role of NRF2 in Mediating Ferroptosis. Cell Chem Biol 27, 436-447 (2020).
81. R. Tian, A. Abarientos, J. Hong, S. H. Hashemi, R. Yan, N. Drager, K. Leng, M. A. Nalls, A. B. Singleton, K. Xu, F. Faghri, M. Kampmann, Genome-wide CRISPRi/a screens in human neurons link lysosomal failure to ferroptosis. Nat Neurosci 24, 1020-1034 (2021).
82. S. C. Sampath, I. Marazzi, K. L. Yap, S. C. Sampath, A. N. Krutchinsky, I. Mecklenbrauker, A. Viale, E. Rudensky, M. M. Zhou, B. T. Chait, A. Tarakhovsky, Methylation of a histone mimic within the histone methyltransferase G9a regulates protein complex assembly. Mol Cell 27, 596-608 (2007).
83. R. Tandon, B. Brandl, N. Baryshnikova, A. Landshammer, L. Steenpass, O. Keminer, O. Pless, F. J. Muller, Generation of two human isogenic iPSC lines from fetal dermal fibroblasts. Stem Cell Res 33, 120-124 (2018).
84. K. J. Brennand, A. Simone, J. Jou, C. Gelboin-Burkhart, N. Tran, S. Sangar, Y. Li, Y. Mu, G. Chen, D. Yu, S. McCarthy, J. Sebat, F. H. Gage, Modelling schizophrenia using human induced pluripotent stem cells. Nature 473, 221-225 (2011).
85. U. Djuric, A. Y. L. Cheung, W. Zhang, R. S. Mok, W. Lai, A. Piekna, J. A. Hendry, P. J. Ross, P. Pasceri, D. S. Kim, M. W. Salter, J. Ellis, MECP2e1 isoform mutation affects the form and function of neurons derived from Rett syndrome patient iPS cells. Neurobiol Dis 76, 37-45 (2015).
86. T. W. Chen, T. J. Wardill, Y. Sun, S. R. Pulver, S. L. Renninger, A. Baohan, E. R. Schreiter, R. A. Kerr, M. B. Orger, V. Jayaraman, L. L. Looger, K. Svoboda, D. S. Kim, Ultrasensitive fluorescent proteins for imaging neuronal activity. Nature 499, 295-300 (2013).
87. H. Cao L. Lia, D Yang, L. Zeng, X. Yewei, B. Yu, G. Liao, J. Chen, Recent progress in histone methyltransferase (G9a) inhibitors as anticancer agents European Journal of Medicinal Chemistry, Vol 179, pages 537-546 (2019).
88. J. R. Haebe, C. J. Bergin, T. Sandouka, and Y. D. Benoit, Emerging role of G9a in cancer stemness and promises as a therapeutic target, Review Oncogenesis, 13;10(11):76, (2021).
89. M.R.C. Charles, A. Mahesh, S.Y. Lin, H.P. Hsieh, A. Dhayalan, M.S. Coumar. Identification of novel quinoline inhibitor for EHMT2/G9a through virtual screening. Biochimie, 168:220-230 (2020).
90. W.N. Pappano, J. Guo, Y. He, D. Ferguson, S. Jagadeeswaran, D.J. Osterling, W. Gao, J.K. Spence, M. Pliushchev, R.F. Sweis, F.G. Buchanan, M.R. Michaelides, A.R. Shoemaker, C. Tse, G.G. Chiang. The Histone Methyltransferase Inhibitor A-366 Uncovers a Role for G9a/GLP in the Epigenetics of Leukemia. PLoS One, 10(7), (2015).

## Claims

1. Compound which is effective in inhibiting the function of the euchromatic histone lysine N-methyltransferase-2 (G9a) for use in a method of treating Multiple Sclerosis or preventing the progression of Multiple Sclerosis in a subject.

2. Compound which is effective in inhibiting the function of the euchromatic histone lysine N-methyltransferase-2 (G9a) for use in a method of reducing or ameliorating ferroptosis in a Multiple Sclerosis patient.

3. Compound for use according to claim 1 or 2, wherein said Multiple Sclerosis is relapsing-remitting Multiple Sclerosis.

4. Compound for use according to any of claims 1 to 3, wherein said subject is a human.

5. Compound for use according to any of claims 1 to 4, wherein the compound is a small molecule having a molecular weight of below 550 Dalton.

6. Compound for use according to any of claims 1 to 5, wherein the compound is selected from the group consisting of UNC0642, UNC0638 and BIX01294.

7. Compound for use according to any of claims 1 to 6, wherein the compound is to be administered in an amount from 1.0 µg/kg body weight to 5,000 µg/kg bodyweight.

8. Pharmaceutical composition comprising a compound which is effective in inhibiting the function of the euchromatic histone lysine N-methyltransferase-2 (G9a) for use in a method of treating Multiple Sclerosis or preventing the progression of Multiple Sclerosis in a subject.

9. Pharmaceutical composition comprising a compound which is effective in inhibiting the function of the euchromatic histone lysine N-methyltransferase-2 (G9a) for use in a method of reducing or ameliorating ferroptosis in a Multiple Sclerosis patient.

10. Pharmaceutical composition for use according to claim 8 or 9, wherein said composition further comprises at least one excipient, diluent, carrier.

11. Pharmaceutical composition for use according to any of claims 8 or 10, wherein said composition comprises at least one additional pharmaceutically active compound which is useful in the treatment of Multiple Sclerosis.

12. Pharmaceutical composition for use according to any of claims 8 or 11, wherein the compound is selected from the group consisting of UNC0642, UNC0638 and BIX01294.

13. Pharmaceutical composition for use according to any of claims 8 or 12, wherein the compound is to be administered in an amount from 1.0 µg/kg body weight to 5,000 µg/kg bodyweight.

14. *In vitro* method for identifying a pharmaceutically active compound for treating Multiple Sclerosis or preventing the progression of Multiple Sclerosis in a subject, comprising
(a) contacting a candidate compound with a functional euchromatic histone lysine N-methyltransferase-2 (G9a);
(b) detecting whether said candidate compound interferes with the function of the euchromatic histone lysine N-methyltransferase-2 (G9a);
wherein a compound which inhibits the function of the euchromatic histone lysine N-methyltransferase-2is (G9a) suitable for treating or preventing the progression of Multiple Sclerosis in a subject.

15. *In vitro* method for identifying a pharmaceutically active compound for treating Multiple Sclerosis or preventing the progression of Multiple Sclerosis in a subject, comprising
(a) contacting a candidate compound with a cell that expresses a functional euchromatic histone lysine N-methyltransferase-2 (G9a);
(b) detecting whether said candidate compound decreases the transcription of the euchromatic histone lysine N-methyltransferase-2 gene and/or the translation of the euchromatic histone lysine N-methyltransferase-2 mRNA;
wherein a compound which decreases the transcription of the euchromatic histone lysine N-methyltransferase-2 gene and/or the translation of the euchromatic histone lysine N-methyltransferase-2 mRNA is suitable for treating or preventing the progression of Multiple Sclerosis.
